(19) Europäisches Patentamt
European Patent Office
Office européen des brevets

(11) EP 1 283 270 A1

(12) **DEMANDE DE BREVET EUROPEEN**

(43) Date de publication:
**12.02.2003 Bulletin 2003/07**

(21) Numéro de dépôt: **02291969.0**

(22) Date de dépôt: **06.08.2002**

(51) Int Cl.[7]: **C12Q 1/00**, G01N 33/543, G01N 27/327, A61L 27/00, A61L 29/00

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **07.08.2001 FR 0110542**

(71) Demandeur: **WARNER-LAMBERT COMPANY**
**Morris Plains, New Jersey 07950 (US)**

(72) Inventeurs:
- **Drexler, Cathy**
**75007 Paris (FR)**
- **Berna, Patrick**
**91120 Palaiseau (FR)**

(74) Mandataire: **Becker, Philippe et al**
**Cabinet Becker & Associés**
**35, rue des Mathurins**
**75008 Paris (FR)**

(54) **Nouvelle membrane supportée, préparation et utilisations**

(57) La présente invention décrit une nouvelle membrane artificielle supportée, ainsi que des méthodes de préparation d'une telle membrane ou de reconstitution de membranes cellulaires à partir des protéines et lipides qui les composent. Cette membrane comprend une bicouche lipidique reliée à un support par des piliers phospholipidiques (P) ménageant un espace entre la bicouche et le support, ainsi qu'un ou plusieurs ligands (L) spécifiques d'une protéine, reliés de manière covalente au support et exposés dans ledit espace. L'invention décrit également les utilisations de ces membranes qui permettent de purifier et/ou de capturer de façon réversible des protéines membranaires ou encore de cribler des composés interagissant avec certaines de ces protéines. Une membrane selon l'invention peut encore servir à déterminer la toxicité ou au contraire l'effet thérapeutique de molécules tests. L'invention est particulièrement utile dans l'analyse des interactions protéine-protéine au sein de la membrane et peut ainsi être utilisée dans l'industrie pharmaceutique pour l'analyse du profil toxique ou bénéfique de molécules qui pourront entrer en développement pharmaceutique et/ou dans des compositions pharmaceutiques. Dans le domaine des biotechnologies ou dans le domaine médical, de telles membranes supportées peuvent aussi être utilisées dans la fabrication de biomatériaux plus performants.

EP 1 283 270 A1

## Description

La présente invention décrit une nouvelle membrane artificielle supportée, ainsi que des méthodes de préparation d'une telle membrane ou de reconstitution de membranes cellulaires à partir des protéines et lipides qui les composent. Cette membrane comprend une bicouche lipidique reliée à un support par des piliers phospholipidiques (P) ménageant un espace entre la bicouche et le support, ainsi qu'un ou plusieurs ligands (L) spécifiques d'une protéine, reliés de manière covalente au support et exposés dans ledit espace. L'invention décrit également les utilisations de ces membranes qui permettent de purifier et/ou de capturer (éventuellement de façon réversible) des protéines membranaires ou encore de cribler des composés interagissant avec certaines de ces protéines. Une membrane selon l'invention peut encore servir à déterminer la toxicité ou au contraire l'effet thérapeutique de molécules tests. L'invention est particulièrement utile dans l'analyse des interactions protéine-protéine au sein de membranes et peut ainsi être utilisée dans l'industrie pharmaceutique pour l'analyse du profil toxique ou bénéfique de molécules qui pourront entrer en développement pharmaceutique et/ou dans des compositions pharmaceutiques. Dans le domaine des biotechnologies ou dans le domaine médical, de telles membranes supportées peuvent aussi être utilisées dans la fabrication de biomatériaux plus performants.

Les membranes cellulaires sont généralement constituées d'une bicouche lipidique dont les éléments sont maintenus en place grâce à des forces intermoléculaires. Ce sont notamment des liaisons hydrophobes qui maintiennent la cohésion de la bicouche. Un lipide est généralement défini comme une molécule constituée d'une chaîne hydrocarbonée hydrophobe et d'un groupe polaire hydrophile. Ces membranes séparent les compartiments cellulaires et sont donc exposées à la fois au compartiment interne et au compartiment externe de la cellule.

Les membranes cellulaires sont le siège de processus de transduction nécessaires à la vie de la cellule. Des récepteurs membranaires spécialisés sont les sites de liaison spécifiques de ligands permettant ainsi la transmission de signaux extra-cellulaires au travers de la membrane, leur amplification et leur intégration. Les bicouches lipidiques supportées font donc d'excellents modèles pour étudier les interactions ligand-récepteurs se produisant à la surface des membranes cellulaires.

Les membranes supportées décrites dans l'art antérieur comportent des bicouches phospholipidiques immobilisées sur des surfaces métalliques, la couche lipidique proximale se trouvant à distance de ladite surface de manière a ménager un espace aqueux au sein duquel des molécules membranaires réceptrices peuvent venir se loger (cf: WO 96/38726). Des molécules espaceurs hydrophiles sont ainsi utilisées pour maintenir cet espace aqueux essentiel au fonctionnement des protéines ancrées dans la membrane et au maintien de la souplesse membranaire. La surface métallique peut être recouverte d'une SAM (Self-Assembled Monolayer), constituée de molécules comprenant de longues chaînes, de préférence hydrocarbonées et dont le nombre d'atomes de carbone est généralement supérieur à 10, qui sont parfois interrompues par des hétéroatomes. Ces chaînes possèdent un groupe fonctionnel permettant l'ancrage de la membrane au support.

L'un des inconvénients essentiels que présentent les membranes artificielles connues est leur inadaptation à toute purification dans la mesure où elles ne permettent pas de retenir ou capturer les protéines membranaires que l'on cherche à purifier dans le but d'enrichir la membrane artificielle en lesdites protéines membranaires.
L'obtention de membranes artificielles enrichies en une espèce moléculaire (e.g. protéine membranaire) s'avère déterminante pour étudier ladite espèce moléculaire dans son environnement naturel de manière sélective et spécifique. Les propriétés de sélectivité et de spécificité sont critiques pour l'utilisation des membranes artificielles dans les tests de criblage concernant des protéines membranaires, à fortiori et en particulier dans les tests de criblage à haut débit de type HTS.
La présente invention permet, notamment grâce à la présence de piliers et de molécules capables de retenir ou capturer l'espèce moléculaire d'intérêt dans sa forme ou conformation naturelle, d'obtenir des membranes supportées enrichies en ladite espèce moléculaire et en particulier des membranes supportées ne comprenant que ladite espèce moléculaire.

D'autre part, les membranes connues (WO 96/38726) imposent une contrainte dans la dimension et la disposition des espaces pouvant accueillir les protéines membranaires. La présente invention permet de moduler l'architecture des espaces pouvant accueillir les protéines membranaires en faisant varier la disposition et la densité des piliers qui supportent la membrane. Il en résulte un meilleur contrôle de la stabilité et de la fluidité de la membrane sur la totalité de la membrane supportée.

La présente invention propose ainsi pour la première fois des méthodes pour la préparation de membranes artificielles comprenant une bicouche lipidique reliée à un support par des piliers phospholipidiques (P) qui ménagent un espace (aqueux) entre la bicouche et le support, et un ou plusieurs ligands (L) spécifiques d'une protéine (ou d'une molécule d'intérêt) reliés de manière covalente au support et exposés dans ledit espace. Ces ligands ont la particularité de renforcer considérablement la spécificité de liaison à un ou plusieurs types spécifiques de protéines d'intérêt.

L'invention décrit ainsi des membranes supportées permettant la fixation spécifique de protéines d'intérêt, dans une configuration fonctionnelle. Cette caractéristique offre de nombreux avantages, comme notamment celui de l'utilisation des membranes pour la purification de protéines à partir de préparations membranaires.
Par « purification de protéines à partir de préparations membranaires », on entend notamment l'enrichissement en une espèce moléculaire d'intérêt d'une préparation membranaire.
La présente demande décrit aussi des conditions de préparation de membranes supportées artificielles, permettant d'assurer une meilleure stabilité et une meilleure flexibilité à la membrane, pour des applications de purification, criblage, etc.
A cet égard, une méthode de l'invention comprend notamment les étapes suivantes :

a) mettre une surface fonctionnalisée en présence d'un mélange de ligands spécifiques (L) et de piliers phospholipidiques (P) susceptibles de se lier à ladite surface fonctionnalisée, et
b) mettre la surface obtenue en a) en présence de lipides de manière à permettre la formation d'une bicouche lipidique supportée par les piliers phospholipidiques (P).

La surface fonctionnalisée comporte généralement des molécules porteuses de groupements fonctionnels permettant de lier, de manière covalente, les piliers phospholipidiques (P) et/ou les ligands spécifiques (L), et des molécules porteuses de groupements non fonctionnels.
L'invention concerne également l'utilisation des membranes supportées définies ci-avant, des kits les comprenant et des procédés de purification ou de criblage de composés les mettant en oeuvre.
Pour une meilleure compréhension des caractéristiques de l'invention et avant d'exposer les méthodes préférées permettant de produire une membrane artificielle supportée, il convient de définir la nature des éléments constituant une telle membrane.

Nature des surfaces

Les surfaces utilisées sont des surfaces artificielles. Parmi les matières utilisables on peut citer notamment les surfaces constituées ou à base de matériaux tels que l'or, le verre, le diamant, le silicium, le dioxyde de silicium ($S_iO_2$), le nitrite de silicone, l'oxyde de tantale ($Ta_2O_5$), le dioxyde de titane($T_iO_2$), le nitrite de titane, le carbide de titane, le platine, le tungstène, l'aluminium ou l'oxyde d'indium/étain, seuls ou en mélanges. On préfère utiliser des surfaces métalliques, comme notamment des surfaces en ou à base d'or.
Un exemple de surface utilisable est une surface d'or commerciale du type Sia Kit Au (Biacore). Ce support est adapté à l'analyse par Surface Plasmon Resonance (SPR) et est compatible avec le BIAcore.
Les surfaces analysées ou utilisées peuvent adopter toutes formes utiles sur le plan fonctionnel et peuvent, par exemple, être des espaces bidimensionnels, de forme carrée, rectangulaire ou circulaire, de préférence plane, ou des espaces tridimensionnels, de formes cubiques, parallélépipédiques ou sphériques. Leurs aires varient entre 1 $mm^2$ et 100 $cm^2$. De manière préférée, la surface est comprise entre 1 et 5 $cm^2$. De manière encore plus préférée, elle est comprise entre 1 et 2,4 $cm^2$.

Description de la surface fonctionnalisée

La surface servant de support est fonctionnalisée, c'est-à-dire de préférence recouverte d'un mélange de molécules porteuses de groupements fonctionnels et non-fonctionnels. De préférence, la surface est fonctionnalisée sous forme de SAM (« Self-Assembled monolayer »). La surface peut être fonctionnalisée avec plusieurs types de groupes fonctionnels capables d'interagir, préférentiellement de manière covalente, avec les groupes (P) et/ou (L).
Certains groupements fonctionnels peuvent en outre être activés de manière à interagir avec le pilier (P) et/ou le ligand (L).
Parmi les groupements fonctionnels utilisés, certains peuvent lier spécifiquement les piliers phospholipidiques (P) tandis que d'autres lient spécifiquement les ligands spécifiques (L). Ce mode de mise en oeuvre permet un contrôle élevé des rapports (P)/(L) dans la membrane. A cet égard, le rapport (P)/(L) est de préférence compris entre 0 et 10, plus préférentiellement entre 0 et 4 et, de manière encore plus préférée, entre 0,01 et 1.
Le type de groupement fonctionnel est choisi en fonction de la réaction de couplage utilisée pour la fixation du pilier (P) et de la molécule spécifique (L). Ces groupements peuvent par exemple correspondre à un groupe COOH, CHO, OH, $NH_{2,}$ maléimide, ou biotine. Après activation, ces groupements forment une liaison covalente de type amide (-NHCO-) ou imide (-CHNH-) si le groupe (P) ou (L) est porteur d'une fonction NH2, d'un pont disulfure (-S-S-) ou d'un thioether (-C-S-) si le groupe (P) ou (L) est porteur d'une liaison - SH ou encore un complexe stable biotine-streptavidine/avidine lorsque le groupe (P) ou (L) est fonctionnalisé par une streptavidine ou une avidine.
L'un des groupes fonctionnels préférés selon l'invention est le groupe COOH. Ce dernier peut faire l'objet d'une activation et donner ainsi lieu à la formation d'un ester par réaction en milieu aqueux avec du N-Hydroxysuccinimide (NHS) et en présence de dicyclohexylcarbodiimide (DCC).

Selon un premier mode particulier de réalisation de l'invention, la surface fonctionnalisée comporte des groupements fonctionnels X éventuellement activables, capables d'immobiliser les ligands spécifiques (L) et les piliers phospholipidiques (P), et des groupements Z non fonctionnels.

Le terme groupe non-fonctionnel désigne toute molécule incapable de réagir (i.e., de lier de manière covalente et spécifique) une molécule (P) ou (L). Il peut s'agir de toute molécule non réactive ou de groupes tels que mentionnés ci-dessus, mais non préalablement activés.

Dans un deuxième mode particulier, la surface est recouverte d'un mélange de molécules comprenant des molécules porteuses de groupements fonctionnels X et Y, éventuellement activables, capables d'immobiliser de manière covalente et spécifique les piliers phospholipidiques (P) et/ou les ligands spécifiques (L).

Dans un autre mode particulier de réalisation de l'invention, la surface fonctionnalisée comporte un mélange de molécules porteuses de groupements fonctionnels X et Y, les groupements X et Y liant sélectivement les ligands spécifiques (L) et les piliers (P), respectivement.

Dans la préparation des membranes de l'invention, le rapport molaire des groupements fonctionnels X et Y sur les groupements non-fonctionnels Z est généralement compris entre 0,05 et 20, de préférence entre 0,1 et 5. De manière particulièrement avantageuse, ce rapport est inférieur à 1 et, plus préférentiellement, compris entre 0,1 et 0,3. Les études réalisées montrent que ces rapports préférés confèrent des propriétés de stabilité suffisantes tout en permettant l'insertion, dans les membranes, de protéines ou complexes protéiques importants.

Les molécules utilisées pour recouvrir la surface peuvent être des dérivés thiol de formules $HS-(CH_2)_n-X$, $HS-(CH_2)_m-Y$ ou $HS-(CH_2)_p-Z$.
L'intérêt d'utiliser un mélange de deux dérivés thiol au moins est de pouvoir contrôler la densité des groupements fonctionnels présents à la surface et de privilégier ainsi la capture de protéine membranaire d'une taille plus ou moins importante, la stabilité de la membrane et sa fluidité.

Les dérivés thiols peuvent varier par la nature de leur chaîne et, comme indiqué précédemment, par le type de groupement fonctionnel. Ainsi la longueur de la chaîne carbonée varie en fonction des nombres n, m et p. De façon générale n, m et p sont compris entre 2 et 15, plus préférentiellement entre 8 et 15 et, de manière préférée, ils sont égaux à 10 ou 11. La longueur de la chaîne peut être ajustée par l'homme du métier.
Il est souhaitable que toutes les molécules porteuses de groupements fonctionnels aient une longueur sensiblement équivalente, de manière à obtenir une surface homogène. De ce fait, il est préféré que les nombres n, m et p soient essentiellement identiques ou similaires, notamment ne diffèrent pas d'un écart supérieur à + ou - 2. On préfère tout particulièrement utiliser des molécules telles que définies ci-dessus, dans lesquelles n, m et p sont des nombres entiers identiques.

Les groupes nucléophiles qui viennent se fixer sur la surface porteuse peuvent être des thiols comme décrit précédemment mais il peut également s'agir d'amines primaires, secondaires ou tertiaires fixées directement ou par l'intermédiaire d'un espaceur dont la longueur est nécessairement plus courte que celle de l'espaceur du pilier phospholipidique (ce dernier sera plus amplement décrit ultérieurement). Outre les amines, il peut encore s'agir de groupes hydrazides ou carboxylates. Dans ces derniers cas, les lipides mis en contact avec cette surface doivent être porteurs de têtes polaires modifiées par des groupes électrophiles tels que des phosphatidyl esters, des esters ou des acides halides ou encore des groupes carbonyles, époxydes ou vinyles. L'avantage des fonctions thiols, pyridyl disulfides, maleimides ou haloacétate est qu'elles donnent lieu à des réactions sélectives. Ces fonctions sont donc préférées dans le cadre de l'invention.

<u>Description des piliers phospholipidiques (P)</u>

Les piliers selon l'invention comprennent préférentiellement trois parties, à savoir un point d'ancrage à la surface fonctionnalisée, un espaceur et un phospholipide.
Les piliers viennent en effet se fixer à la SAM au niveau des groupements fonctionnels (activés à cet effet), décrits ci-dessus. Comme indiqué, le point d'ancrage comporte un groupe réactif capable de réagir avec un groupe fonctionnel de la surface fonctionnalisée. Un tel groupe fonctionnel peut être par exemple un groupe COOH, CHO, OH, $NH_{2,}$ maléimide ou biotine.

Un objet de la présente invention concerne donc des méthodes particulièrement avantageuses dans lesquelles le pilier phospholipidique (P) comprend un point d'ancrage à la surface fonctionnalisée, un espaceur, partie située entre le point d'ancrage et la tête polaire du phospholipide, et un phospholipide. L'espaceur correspond le plus souvent à une chaîne hydrophile comprenant de 2 à 200 atomes de carbones, la longueur de l'espaceur étant inférieure à 500 Å, préférentiellement comprise entre 3 et 500 Å. A titre d'exemples, on peut citer des espaceurs ayant une longueur de 3.5-5 Å, 11-14 Å ou 170-220 Å, environ.

De manière à obtenir une surface plane, les piliers doivent être de longueurs sensiblement équivalentes.

Le pilier phospholipidique peut être utilisé sous plusieurs formes :

- Il peut s'agir d'une solution aqueuse du phospholipide lorsque sa solubilité dans l'eau est suffisante
- Il peut encore s'agir d'une solution micellaire (mélange lipide/détergent) ou de liposomes lorsque le phospholipide est insoluble en milieu aqueux.

La tête phospholipidique du pilier sert d'amorce pour la fixation de la bicouche. Elle peut correspondre à différents phospholipides, tels que les phospholipides rencontrés dans les membranes des cellules de mammifères ou des cellules végétales ou bactériennes, etc. Il peut par exemple s'agir de la phosphatidyl choline (PC), de la phosphatidyl sérine (PS), de la phosphatidyl éthanolamine (PE), du phosphatityl inositol (PI), du phosphatidyl glycérol (PG), etc.

Différents piliers phospholipidiques commerciaux peuvent être utilisés dans l'invention, qui diffèrent les un des autres par la longueur (I) de l'espaceur et la structure de la chaîne grasse du phospholipide. Parmi les piliers phospholipidiques commerciaux pouvant être utilisés dans le cadre de la présente invention, on trouve notamment le PE, comportant un espaceur dont la longueur peut varier entre 3 et 5 Å, le DOPE-C comportant un espaceur dont la longueur varie entre 11 et 14 Å, le PEG-DPPE comportant un espaceur dont la longueur varie entre 176 et 214 Å (Figure 2).

<u>Description des ligands spécifiques (L)</u>

Le ligand spécifique (L) comprend un groupement d'affinité spécifique pour une molécule particulière et un point d'ancrage à la surface fonctionnalisée. Dans un mode de réalisation particulier, le ligand comprend en outre un espaceur. L'espaceur du ligand peut correspondre à une chaîne hydrophile constituée de 2 à 200 atomes et est choisi de préférence de sorte que la longueur du ligand spécifique soit inférieure à celle du pilier phospholipidique.

Le groupe d'affinité du ligand (L) peut être un produit naturel, synthétique ou chimique présentant une affinité pour une molécule d'intérêt, tel que notamment une protéine et plus particulièrement une protéine membranaire, un récepteur membranaire (capable de se lier à un ligand inhibiteur ou effecteur), une immunoglobuline, une lectine, un antigène, un haptène ou un substrat, etc., ces molécules étant capables de se lier spécifiquement avec un ligand respectif. Selon un mode préféré de l'invention, le ligand spécifique L correspond à un ligand d'affinité spécifique pour un constituant protéique. Il s'agit de façon avantageuse d'un anticorps ou d'un fragment d'anticorps spécifique (e.g., Fab, Fab'2, CRD, ScFv, etc.), d'une protéine ou d'un complexe protéique, notamment d'une partie extramembranaire au moins d'une protéine ou d'un complexe protéique.
Le ligand peut également être une protéine connue pour son affinité spécifique pour une classe de molécule telle qu'une lectine, la streptavidine, la protéine A, la protéine G ou plus largement pour une protéine faisant partie d'un complexe multiprotéique. Selon un autre mode préféré, L correspond à une molécule chimique de faible masse moléculaire présentant une affinité pour la protéine d'intérêt à immobiliser. Cette molécule chimique peut être un inhibiteur, activateur, agoniste, antagoniste, effecteur ou cofacteur de cette protéine.
Une protéine membranaire présente dans une vésicule naturelle ou dans des fragments de membranes peut ainsi être immobilisée sur le support, selon un procédé particulièrement avantageux, par réaction avec le ligand spécifique. La protéine membranaire immobilisée peut être purifiée en introduisant un flux de phospholipide au niveau de la surface de la membrane de sorte que les protéines non liées sont évacuées ou entraînées.

Par ailleurs, il est possible d'utiliser, dans la construction d'une membrane selon l'invention, plusieurs Ligands (L) présentant une groupe d'affinité pour des molécules d'intérêt différentes. Il peut s'agir de deux récepteurs membranaires, d'un récepteur et d'un co-récepteur, d'un récepteur et d'une protéine G, de plusieurs sous-unités d'un récepteur, etc.

Les ligands spécifiques (L) sont fixés à la SAM au niveau des groupements fonctionnels décrits ci-dessus. Comme indiqué, le point d'ancrage comporte un groupe réactif capable de réagir avec un groupe fonctionnel de la surface fonctionnalisée. Lorsque le groupe fonctionnel est un groupe COOH, CHO, OH, $NH_2$, maléimide ou biotine, le groupe réactif peut être un groupe SH, NH2, avidine ou streptavidine, par exemple.

L'espaceur du ligand spécifique (L) peut correspondre également à une chaîne hydrophile. La longueur de l'espaceur est choisie de telle sorte que la longueur du ligand spécifique (L) soit inférieure à celle du pilier (P). De préférence, l'espaceur comprend de 2 à 50 atomes. La longueur de l'espaceur est avantageusement inférieure à 500 Å et est préférentiellement comprise entre 3 et 500 Å et encore plus préférentiellement entre 3 et 400. A titre d'exemples, on peut citer des espaceurs de longueur comprise entre 3 et 5 Å, entre 10 et 20 Å ou entre 150 et 220 Å. La longueur de l'espaceur du ligand (L) est préférentiellement inférieure à la longueur de l'espaceur du pilier (P).

<u>Principe de la réaction de couplage</u>

Le couplage peut être réalisé à l'aide de techniques connues en elles-mêmes, comprenant généralement les étapes suivantes :

- une première étape, facultative, d'activation de la surface fonctionnalisée,
- une étape de couplage des groupements fonctionnels aux piliers (P) et aux ligands spécifiques (L), et
- une étape facultative de désactivation des groupements fonctionnels n'ayant pas réagi.

Les groupements X et/ou Y de la surface fonctionnalisée sont éventuellement activés avant la fixation des piliers et ligands. L'activation du groupement fonctionnel n'est pas systématiquement nécessaire et dépend de sa nature. Si l'on prend comme exemple le groupe COOH, l'activation consiste à faire réagir ce groupe fonctionnel en milieu aqueux et en présence de dicyclohexylcarbodiimide (DCC) avec du N-Hydroxysuccinimide (NHS), de manière à former un ester. X est alors capable de fixer L ou P indifféremment.
Le groupe Y peut alors correspondre à un groupe hydroxyle (OH) non fonctionnel qui n'interviendra pas dans la réaction de couplage.
Une autre possibilité proposée par l'invention consiste à activer à la fois X et Y et à introduire dans le mélange une molécule porteuse d'un groupement non fonctionnel Z, incapable de réagir de manière sélective et covalente avec une molécule (L) ou (P). L'intérêt d'introduire une molécule porteuse d'un groupement non fonctionnel est de préserver des espaces libres entre les piliers phospholipidiques, où pourront venir s'insérer, en se liant aux ligands spécifiques (L), des protéines d'intérêt.
L'étape de couplage s'effectue en une ou deux étapes :
Il peut y avoir co-fixation de P et L, c'est-à-dire fixation simultanée, ou fixation en deux étapes successives de P et de L au niveau de groupements fonctionnels spécifiques à chacun.
Le nombre de molécules L ou P immobilisées à la surface du support est fonction soit des quantités de (P) et (L) utilisées, lorsque le couplage s'effectue par des groupes fonctionnels spécifiques, soit du rapport molaire de (L) et (P) présent dans le mélange, soit de la concentration des groupements fonctionnels pouvant réagir spécifiquement avec (L) et (P).

- Le cas où X correspond au groupe fonctionnel et Z au groupe non fonctionnel peut être représenté par la formule suivante :

$$aX + bZ \rightarrow aX^* + bZ \rightarrow (a-c)P + cL + bZ$$

avec a= nombre de groupements X, X*= groupement X activé, b= nombre de groupements Z, c= nombre de ligands, (a-c)= nombre de piliers (P) et a+b=100.

- Le cas où les groupements fonctionnels X sont activés puis mis successivement en présence du pilier (P) et du ligand spécifique (L) permet un meilleur contrôle de la densité du pilier fixé à la surface de la SAM et peut être représenté par la formule suivante :

$$aX + bZ \rightarrow aX^* + bZ \rightarrow (a-c)P + cX^* + bZ \rightarrow (a-c)P + cL + bZ$$

- Le cas où les groupements fonctionnels X sont activés et réagissent avec le pilier (P) peut être représenté par la formule suivante, les groupements X n'ayant pas réagi étant désactivés à l'aide d'une molécule capable de se lier avec un ligand spécifique (L). :

$$aX + bZ \rightarrow aX^* + bZ \rightarrow (a-x)P + xX^* + bZ$$

$$(a-x)P + xX^* + bZ \rightarrow (a-x)P + xX' + bZ \rightarrow (a-x)P + xL + bZ$$

- Le cas où les groupements X et Y après activation réagissent avec le pilier (P) et le ligand (L) respectivement peuvent être représentés par la formule suivante :

$$aX + bY \rightarrow aX^* + bY \rightarrow aP + bY \rightarrow aP + bY^* \rightarrow aP + bL$$

Les groupements activés n'ayant pas réagi subissent une réaction de désactivation. Dans le cas des groupements carboxylates, de l'éthanolamine (Biacore) peut être utilisée comme moyen de désactivation.

Origine et contenu de la bicouche lipidique

La bicouche lipidique des membranes de l'invention peut comporter des lipides d'origine et de nature variée. Ces lipides peuvent provenir d'extraits cellulaires, de fragments membranaires, de monocouches ou de bicouches lipidiques, de liposomes ou de vésicules lipidiques et/ou de lipides, phospholipides, lipopeptides, lipides biotinylés et leurs mélanges. La bicouche est formée par assemblage des lipides avec les lipides du pilier (P). La quantité de lipides utilisée, quelle que soit la forme sous laquelle ils se présentent, est adaptée par l'homme du métier.
Les membranes planes et continues préparées selon l'invention peuvent être fabriquées à l'intérieur d'un appareil tel que le Biacore afin de maintenir un flux constant de solvant et/ou de soluté et/ou de vésicules lipidiques à la surface de la membrane. Le principe permet de réduire les risques de contamination et de dénaturation ou destruction de la membrane.
De manière préférée, la construction de la membrane est envisagée selon deux méthodes qui consistent en une dilution de micelles ou en une fusion de vésicules.

Méthodes permettant de produire une membrane supportée

La présente invention décrit plusieurs méthodes permettant de produire une membrane supportée. De manière générale, elles comprennent les étapes suivantes :

a) mettre une surface fonctionnalisée en présence d'un mélange de ligands spécifiques (L) et de piliers phospholipidiques (P), susceptibles de se lier à ladite surface fonctionnalisée, et
b) mettre la surface obtenue en a) en présence de lipides de manière à permettre la formation d'une bicouche lipidique supportée par les piliers phospholipidiques (P).

Dans cette méthode, on distingue parmi les groupements fonctionnels les groupements X éventuellement activables, capables d'immobiliser les ligands spécifiques (L) et/ou les piliers phospholipidiques (P), ainsi que les groupements non fonctionnels Z.
Selon un autre mode de réalisation de l'invention, des groupements fonctionnels X et Y peuvent être tous deux activés de manière à pouvoir lier (immobiliser) de façon spécifique les ligands spécifiques (L) et/ou les piliers phospholipidiques (P).
Selon une méthode particulière, la surface comporte un mélange de molécules porteuses de groupements fonctionnels X et Y, les groupements X et Y liant sélectivement les ligands spécifiques (L) et les piliers (P), respectivement.
Comme expliqué précédemment, les molécules porteuses de groupements fonctionnels peuvent être des dérivés thiols de formule HS-$(CH_2)$n-X ou HS-$(CH_2)$m-Y, n et m étant compris entre 2 et 15.
Une autre méthode selon l'invention permet de produire une membrane supportée. Elle comprend les étapes suivantes :

a) recouvrir une surface de molécules de formule HS-$(CH_2)$n-X et HS-$(CH_2)$m-Z, X étant un groupement fonctionnel préalablement activé capable d'immobiliser un ligand spécifique (L) ou un pilier phospholipidique (P), Z étant un groupement non-fonctionnel, n et m étant un nombre entiercompris entre 2 et 15,
b) mettre la surface obtenue en a) en présence d'un mélange contenant les ligands spécifiques (L) et les piliers phospholipidiques (P), puis
c) mettre la surface obtenue en b) en présence de lipides de manière à permettre la formation d'une bicouche lipidique supportée par les piliers phospholipidiques (P).

Selon une autre méthode de l'invention, il est possible de produire une membrane supportée comprenant les étapes suivantes :

a) recouvrir une surface de molécules porteuses de groupements fonctionnels de formules HS-$(CH_2)$n-X et HS-$(CH_2)$m-Z, X étant un groupement fonctionnel préalablement activé capable d'immobiliser un ligand spécifique (L) ou un pilier phospholipidique (P), Z étant un groupement non-fonctionnel, n et m étant compris entre 2 et 15,
b) mettre la surface obtenue en a) en présence des piliers phospholipidiques (P), puis
c) mettre la surface obtenue en b) en présence des ligands spécifiques (L), et
d) mettre la surface obtenue en c) en présence de lipides de manière à permettre la formation d'une bicouche lipidique supportée par les piliers phospholipidiques (P).

Une autre méthode comprend les étapes suivantes :

a) recouvrir une surface au moyen de molécules porteuses de groupements non fonctionnels et de molécules porteuses de groupements fonctionnels de formules HS-$(CH_2)$n-X, HS-$(CH_2)$m-Y et HS-$(CH_2)$n-Z, X et Y étant des groupements fonctionnels préalablement activés capables d'immobiliser respectivement un ligand spécifique (L) et un pilier phospholipidique (P), Z étant un groupement non-fonctionnel et n et m étant compris entre 2 et 15,
b) mettre la surface obtenue en a) en présence d'un mélange contenant les ligands spécifiques (L) et les piliers phospholipidiques (P), puis
c) mettre la surface obtenue en b) en présence de lipides de manière à permettre la formation d'une bicouche lipidique supportée par les piliers phospholipidiques (P)..

Les méthodes décrites ci-dessus peuvent comprendre une étape supplémentaire suivant l'étape a), de désactivation des groupements X et Y n'ayant pas réagi avec les ligands (L) ou les piliers (P). Cette désactivation peut se faire par toute technique connue, fonction de la nature du groupe X ou Y, et par exemple à l'aide de molécules capables de se lier aux groupes X ou Y.
Par ailleurs, dans un mode particulier, le procédé comprend une étape supplémentaire d'introduction d'une molécule d'intérêt dans la membrane. Cette étape peut être réalisée de manière simultanée à la formation de la bicouche, ou postérieurement à celle-ci. Lorsque cette étape est réalisée postérieurement à la formation de la bicouche, elle est réalisée préférentiellement par fusion d'une vésicule naturelle contenant la protéine d'intérêt avec la bicouche préformée.
L'invention englobe toute membrane artificielle obtenue selon l'une quelconque des méthodes exposées précédemment.

<u>Applications des membranes supportées</u>

Les membranes selon l'invention constituent donc un modèle de choix pour étudier les échanges entre l'intérieur et l'extérieur de cellules, sans avoir recours à l'expérimentation animale mais en restant proche de la situation *in vivo.* Elles permettent notamment l'analyse des interactions protéine-protéine au sein de la membrane, en utilisant des protéines purifiées, dans des conditions physiologiques.
L'analyse par SPR est une méthode d'optique qui permet, tout comme l'Ellipsometry and evanescent wave spectrosocy (EWS), la Brewster angle refractometry, la critical angle refractometry, la Frustrated Total Reflexion (FTR), l'evanescent wave ellipsometry, la Scattered Total Internal Reflexion (STIR), etc. de mesurer les concentrations en surface ou l'indice de réfraction. Ces données varient en effet en même temps que la composition de la membrane et permettent ainsi de l'étudier.
Les membranes supportées selon l'invention peuvent être avantageusement utilisées pour la capture réversible de protéines membranaires complexées ou non, à l'aide de leurs ligands spécifiques.
Dans ce contexte, les caractéristiques particulières des membranes artificielles selon l'invention ont permis la mise au point d'un procédé de purification de protéines comprenant (a) la mise en contact d'une membrane supportée décrite ci-avant et/ou obtenue selon l'une quelconque des méthodes de l'invention avec une préparation membranaire comprenant une protéine à purifier, dans des conditions permettant l'introduction de ladite protéine à purifier dans ladite membrane et, (b), l'application d'un flux de lipides. Ce procédé est bien évidemment utilisable pour purifier toute autre molécule contenue dans un extrait membranaire. Ce procédé repose notamment sur l'emploi d'une membrane selon l'invention comprenant un ligand spécifique (L) de ladite molécule.
Les protéines transmembranaires sont immobilisées de façon directe ou indirecte dans une bicouche lipidique par l'intermédiaire de la molécule spécifique (anticorps, protéine d'interaction, substrats, inhibiteurs...) que constitue le ligand spécifique (L). Les protéines qui n'ont pas été fixées peuvent être éluées à l'aide d'un flux de phospholipides sous la forme de micelles ou de liposomes. Cette technique constitue un moyen efficace pour purifier en condition douce non dénaturante des protéines transmembranaires.
Les protéines transmembranaires ainsi purifiées peuvent ensuite permettre de développer des tests de criblage de molécules actives à partir des récepteurs membranaires. La présente invention inclut donc l'utilisation d'une membrane supportée obtenue selon l'une quelconque des méthodes de l'invention, pour le criblage de composés interagissant avec des protéines membranaires natives incluses dans ladite membrane supportée.
Les membranes supportées selon l'invention qui sont enrichies en un ou plusieurs types particuliers de protéines peuvent être utilisées notamment dans la fabrication des biocapteurs capables de convertir une activité biologique en un signal quantifiable. Les biomatériels ainsi utilisés sont appelés à être mis en contact avec les fluides et tissus corporels et doivent donc satisfaire à des critères de compatibilité. Les protéines et cellules sont en effet connues pour s'adsorber à la surface des matériels artificiels lorsque ces derniers sont mis en contact avec le sang ou d'autres fluides biologiques ce qui provoque des effets indésirables. L'existence de membrane s'intégrant dans ce contexte physiologique particulier permettrait d'éviter ou de minimiser ces effets indésirables.
Dans un mode particulier de mise en oeuvre des méthodes d'étude des tissus reconstitués de l'invention, ces derniers peuvent être traités par une ou plusieurs molécule test de façon à caractériser les réactions membranaires à l'égard de cette ou ces molécules test.

La molécule test peut être de nature très variée. Ainsi, il peut s'agir d'une molécule isolée ou d'un mélange de substances. Le composé peut être de nature chimique ou biologique. Il peut s'agir notamment d'un peptide, polypeptide, acide nucléique, lipide, glucide, d'une molécule chimique, d'extraits végétaux, de banques combinatoires, etc. Pour la mise en oeuvre du procédé de l'invention, le composé test peut être appliqué à différentes concentrations, choisies par l'utilisateur.

La présente invention a donc pour objet l'utilisation d'une membrane reconstituée telle que définie ci-avant, pour l'évaluation des propriétés biologiques et/ou toxiques d'une molécule test, notamment d'une molécule à visée thérapeutique, cosmétique, etc.

Ce procédé d'étude des membranes reconstituées peut également permettre l'analyse des transcrits des différents types cellulaires ainsi que l'analyse de l'activité électrique, l'étude des canaux ioniques impliqués dans les échanges et/ou celle des récepteurs, par des techniques électrophysiologiques ou par des techniques de patch-clamp.

La faisabilité, la réalisation et d'autres avantages de l'invention sont illustrés plus en détails dans les exemples qui suivent, qui doivent être considérés comme illustratifs et non limitatifs.

**Légende des figures :**

Figure 1: Procédure de fixation des dérivés thiol (incubation dans une solution d'éthanol à 1mM à 4°C pendant 16 heures ; rinçage à l'aide d'une solution d'éthanol, sonicate pendant 1 min et à deux reprises ; fixation sur la surface en or du support Biacore).

Figure 2 : Structure de Piliers phospholipidiques.

Figure 3 : Exemple de pilier phospholipidique (POPC). Une solution micellaire contenant 2 mg de POPC dans 1 ml d'une solution à 50 mM d'octylβ-D-glucoside (OG) et dans un milieu tamponné HBS-N (0.01 M HEPES, 0.15 M NaCl, pH 7.4) est mise en présence de la surface fonctionnelle.

Figure 4 : Résultats obtenus pour chacune des surfaces utilisées mises en présence d'un réactif.

(I) est l'intensité du signal en RU avant l'injection d'un réactif et (F) après injection, la variation (Δ) du signal correspond à la différence (F-I). Les intensités sont indiquées pour l'injection de BSA avant la formation de membrane (injection 2), de micelles de POPC (injection 4), de NaOH (injection 5 et 6), de BSA après formation de la membrane de POPC (injection 7) et de BSA après régénération de la surface (injection 9).

Figure 5 :

a) Synthèse de la membrane POPC sur Sia-0.
b) Synthèse de la membrane POPC sur Sia-11.
c) Synthèse de la membrane POPC sur Sia-20.
d) Synthèse de la membrane POPC sur Sia-33.
e) Synthèse de la membrane POPC sur Sia-70.
f) Synthèse de la membrane POPC sur Sia-100.

Figure 6 : Effet du pH sur l'étape d'immobilisation.

Figure 7 : Effet du pH sur l'étape d'immobilisation.

Figure 8 : Immobilisation du lipide DOPE-C sur différentes surfaces.

Figure 9 : Immobilisation du lipide PEG-PE sur différentes surfaces.

Figure 10 : Suivi en RU des étapes de formation d'une membrane supportée.

**Matériels et Méthodes**

Dans cet exemple une Surface d'or commerciale Sia Kit Au (Biacore) a été utilisée.

Six supports différents (Sia-100, -70, -33, -20, -11, -01) ont été préparés par réaction spontanée d'une solution d'éthanol 1 mM contenant le mélange de deux dérivés thiols commerciaux utilisés à différentes fractions molaires avec la surface d'or (Figure n° 1).

Les dérivés thiols concernés sont :

- L'acide 11-mercaptoundecanoique (HS-(CH2)10-COOH)
- Le 11-mercapto-1-undecanol (HS-(CH2)11-OH)

Intérêt du choix des dérivés thiol mentionnés ci-dessus : les groupements fonctionnels -COOH servent de point d'attache du pilier. En faisant varier la concentration des groupements -COOH exposés à la surface des SAM, la densité des piliers peut être contrôlée.

Les méthodes de couplage covalent de molécules sur le support fonctionnalisé à l'aide d'une SAM s'apparentent à celles généralement utilisées pour la préparation d'une matrice de chromatographie d'affinité. La réaction de couplage peut être envisagée à l'intérieur de l'appareil Biacore ou à l'extérieur de l'appareil.

Dans notre cas, le couplage du pilier avec le groupement -COOH a été réalisé à l'intérieur. Cela permet de suivre l'efficacité de la réaction de couplage en fonction de l'intensité du signal en RU (unité Biacore).

La fixation du pilier est réalisée par l'intermédiaire d'une liaison covalente de type amide :

a) Le groupement -COOH est activé sous la forme d'un ester par réaction en milieu aqueux avec du N-Hydroxysuccinimide (NHS) et en présence de dicyclohexylcarbodiimide (DCC).

b) Le pilier phospholipidique réagissant avec l'ester activé peut être utilisé de plusieurs façons :

- une solution aqueuse du phospholipide lorsque sa solubilité dans l'eau est suffisante,
  et pour des phospholipide insoluble en milieu aqueux :

- sous la forme d'une solution micellaire (mélange lipide/détergent),
- sous la forme de liposomes.

La préparation de micelles nécessite l'emploi de détergent. Notre choix s'est basé sur l'octyl glucoside (OG), détergent non ionique qui est facilement évacué par un lavage à l'eau (cmc élevée), et qui présente dans sa structure des groupements -OH peu réactifs vis à vis de l'ester activé.
Les structures des différents phospholipides sont représentées sur la figure 2. Ces phospholipides sont des produits commerciaux. Ils se différencient par la présence ou non d'un espaceur et la nature de la chaîne aliphatique (longueur, nombre d'insaturation). Le point de jonction entre l'espaceur et la tête polaire du phospholipide est une jonction de type amide. L'allongement de l'espaceur peut être envisagé par une fixation successive via une liaison de type amine de fragments (Figure n°3 ).

c) Les groupements carboxylates qui n'ont pas réagi avec le phospholipide sont désactivés en présence d'éthanolamine. La synthèse de membrane sur différents supports utilisant les lipides ci-dessus est représentée sur la figure 5.

**<u>Exemple n°1 (Figures 6 et 7)</u>**

Différentes conditions de pH ont été testées afin d'optimiser l'étape d'immobilisation du phospholipide (Figure 6).
La procédure décrite suivante a été mise en oeuvre, de manière entièrement automatisée :

| | | |
|---|---|---|
| 1. | Détergent OG 50 mM dans un tampon X | (100µl ; 10 mn) |
| 2. | NaOH 50mM | (10µl ; 1 mn) |
| 3. | NaOH 50mM | (10µl ; 1 mn) |
| 4. | EDC/NHS | (100µl ; 10 mn) |
| 5. | Lipides (2 mg/ml de OG 50 mM dans du tampon X) | (100µl ; 20 mn) |
| 6. | Lipides (2 mg/ml de OG 50 mM dans du tampon X) | (100µl ; 20 mn) |
| 7. | Détergent OG 50 mM dans du tampon X | (10µl ; 1 mn) |
| 8. | Ethanolamine | (60µl ; 6 mn) |
| 9. | Détergent OG 50 mM dans du tampon X | (10µl ; 10 mn) |
| 10. | NaOH 50 mM | (10µl ; 1 mn) |

La surface est conditionnée (injection1-3), activée (4), le lipide est couplé (injection 5 et 6), les lipides qui n'ont pas été fixés sont évacués par une injection de détergeant (7) puis les groupements carboxylates sont désactivés (8). Une nouvelle injection de OG permet d'évacuer les molécules d'éthanolamine adsorbées.

Les valeurs de pH testés se situent entre 7.5 et 11 et sont établies à partir de tampon phosphate (PB) ou Borate-NaOH (BB) 0.1 M. Le phospholipide DOPE-C est injecté sous la forme d'une solution micellaire (2mg/ ml de OG 50 mM) dans les différents tampons sur la surface Sia-100 (100% de groupements COOH). Le graphe 1 indique l'intensité du signal en RU avant et après chaque injection de réactif. Les intensités sont relatives car les signaux 1 et 4 obtenus avant l'injection sont pris arbitrairement comme référence et donc égaux à 0. Le taux de fixation augmente progressivement jusqu'à atteindre un maximum à pH 10. Des résultats analogues ont été obtenus à partir du lipide DPPE-C (Figure 7).

**Exemple n°2**

Dans le cadre d'un deuxième exemple, le lipide DOPE est immobilisé à pH 10 sur les 6 surfaces présentant une concentration variable de groupements COOH en suivant la procédure décrite dans l'exemple 1. Les résultats obtenus sont présentés sur la Figure 8.

La figure 8 indique l'intensité du signal en RU observé avant l'étape de désactivation (injection 8) pour l'ensemble des surfaces Sia-0, -11, -20, -33, - 70, -100%. La réaction d'immobilisation est réalisée sur deux canaux différents (fc=1, fc=2) au niveau d'une même surface.

On observe pour fc=1 et fc=2 un taux de lipide DOPE-C immobilisé semblable pour l'ensemble des surfaces, mis à part pour Sia-70%. Le taux d'immobilisation est maximum pour Sia-100% puis diminue de moitié pour Sia-70% pour atteindre une valeur minimum observée pour Sia-33, -20, -11 et Sia-0 %.

Expériences témoins :

En présence de DOPE-C, le signal observé pour Sia-0% qui ne présente pas de groupement COOH activable peut être due à une intercalation du phospholipide. En effet, aucun signal n'est observé lorsque seul le détergent OG est injecté. Dans le cas de Sia-100%, le signal peut être du à une dérive de la ligne de base au cours de l'expérience. Dans le cas d'une fixation partielle du détergent, la méthode qui consiste à injecter une solution contenant des liposomes du lipide à immobiliser permet de contourner le problème.

**Exemple n°3**

La même expérience que l'exemple 2 a été réalisée à partir du phospholipide PEG-PE (PM 2739.37 g/mol) en suivant la procédure décrite ci-dessous :

| | |
|---|---|
| NaOH 50 mM | 10 µl (1min) |
| NaOH 50 mM | 10 µl (1min) |
| EDC/NHS | 100 µl (10 min) |
| PEG-PE (5 mg/ml dans tampon BB, pH10) | 50 µl (10 min) x 3 |
| Ethanolamine | 60 µl (6min) |
| NaOH 50 mM | 10 µl (1min) |
| NaOH 50 mM | 10 µl (1min) |

L'emploi du détergent OG n'a pas été nécessaire car le phospholipide PEG-PE est soluble dans l'eau. Les molécules de PEG-PE qui n'ont pas été fixées de façon covalente sont évacuées à l'aide du tampon de course HBS-N.

La Figure 9 indique le taux de lipide PEG-PE immobilisé, i.e l'intensité du signal en RU observé avant l'étape de désactivation (étape 8) pour l'ensemble des surfaces Sia-0, -11, -20, -33, -70, -100%. La réaction d'immobilisation est réalisée sur deux canaux différents (fc=1, fc=2) au niveau d'une même surface.

On obtient également pour fc=1 et fc=2 un taux de lipide PEG-PE immobilisé semblable pour l'ensemble des surfaces. Le taux d'immobilisation diminue en fonction du pourcentage de groupements fonctionnels -COOH exposés au niveau de la surface. La diminution est globalement faible (valeur maximum ≅1800 RU, minimum ≅ 1200 RU). Les variations les plus significatives sont obtenues pour les surfaces Sia-33, sia-20, sia-11%.

Lorsque l'expérience est réalisée à partir de la surface fonctionnalisée uniquement avec des groupements hydroxyles -OH non activables, on trouve un taux moyen de PEG-PE immobilisé égal à 718 RU. Lorsque l'activateur EDC/NHS (injection 3) est remplacé par une simple injection de tampon HBS-N, on obtient un signal de 570 RU. Les groupements -OH ne réagissant pas en théorie avec l'activateur, le signal observé pourrait donc correspondre à une intercalation du phospholipide au niveau de la SAM et/ou une dérive de la ligne de base au cours de l'expérience.

**Exemple 4 : Formation d'une membrane**

La construction de la membrane peut être envisagée selon deux méthodes :

- celle impliquant la dilution de micelles
- celle impliquant la fusion de vésicules

La méthode de dilution de vésicules a été explorée dans le cadre de cet exemple.
La surface est régénérée par une simple injection de détergent OG.

Le lipide POPC, constituant membranaire, est injecté sous la forme d'une solution micellaire (2mg/ml de OG 50 mM dans le tampon HBS-N).

La procédure mise au point pour la formation de la membrane est entièrement automatisée :

| | | |
|---|---|---|
| 1. | Détergent OG 50 mM dans du tampon HBS-N | (100µl ; 10 mn) |
| 2. | BSA (0.1 mg/ml) | (50µl ; 10 mn) |
| 3. | Détergent OG 50 mM dans du tampon HBS-N | (100µl ; 10 mn) |
| 4. | Micelles | |
| | 2 mg de lipides/ml issus de OG 50 mM dans HBS-N | (100µl ; 20 mn) |
| | 1 mg/ml issu de OG 25 mM dans HBS-N | (50µl ; 10 mn) |
| 5. | NaOH 20 mM | (10µl ; 1 mn) |
| 6. | NaOH 20 mM | (10µl ; 1 mn) |
| 7. | BSA (0.1 mg/ml) | (50µl ; 10 mn) |
| 8. | Détergent OG 50 mM dans du tampon HBS-N | (100µl ; 10 mn) |
| 9. | BSA (0.1 mg/ml) | (50µl ; 10 mn) |
| 10. | Détergent OG 50 mM dans du tampon HBS-N | (100µl ; 10 mn) |

L'étape de dilution consiste à injecter successivement une solution micellaire du lipide POPC (2mg/ml de OG 50 mM dans le tampon HBS-N) puis la solution diluée au ½ dans du tampon HBS-N.
L'injection de BSA est utilisée comme marqueur de formation d'une bicouche. En effet, la BSA se fixe préférentiellement sur des surfaces hydrophobes (e.g., surface ne présentant pas de membrane), i.e. avant injection de POPC ou après régénération de la surface. Un signal < 100 RU devrait être observé en présence de membrane.

Le tableau représenté sur la figure n°4 regroupe les résultats obtenus pour chacune des surfaces. (I) est l'intensité du signal en RU avant l'injection d'un réactif et (F) après injection, la variation (Δ) du signal correspond à la différence (F-I). Les intensités sont indiquées pour l'injection de BSA avant la formation de membrane (injection 2), de micelles de POPC (injection 4), de NaOH (injection 5 et 6), de BSA après formation de la membrane de POPC (injection 7) et de BSA après régénération de la surface (injection 9).

Le signal correspondant à la formation de la membrane de POPC est obtenu après avoir effectué un lavage de la surface à l'aide d'une solution de NaOH 20 mM (injection 5 et 6) suivi d'un retour à un signal stable, i.e. colonne (I) dans BSA (7). Les intensités des signaux correspondant à la formation de membrane de POPC sont représentées pour chaque surface en fonction du taux de lipides DOPE-C greffés sur les différents canaux.

On observe que BSA (7) est < 100 RU. BSA (2) et (9) sont supérieurs à BSA (7).
Sia-100 et -0% : l'intensité du signal après formation de membrane est comparable en présence ou en absence de pilier phospholipidique. Elle est égale à 700 et 1200 RU en moyenne et respectivement.

Sia-11, -20, -33 et -70 % : l'augmentation du signal après formation de membrane de POPC est plus importante en présence de pilier (P) de DOPE-C qu'en absence de pilier. En présence de pilier, l'augmentation du signal correspondant à la formation de membrane de POPC est indépendante de la densité de piliers. On obtient en moyenne une variation de + 1000 à 1200 RU.

**Exemple n° 5 : Test d'immobilisation de protéine membranaire**

Des fragments de membranes provenant de cellules CHO où le récepteur R-CGRP couplé à la protéine Ramp est surexprimé ont été déposés sur la surface Sia-11%. La réaction est conduite in-situ dans le BIAcore.

**Exemple n° 6 : densité des piliers**

Le calcul de la densité du pilier DOPE-C (en RU) sur la surface des SAM est réalisé en considérant qu'une chaîne carbonée occupe une surface de 0.40 $nm^2$, qu'une molécule de dérivé thiol HS- (CH2)11-OH ou HS - (CH2) 10-COOH occupe 0.40 $nm^2$ et qu'une molécule de phospholipide DOPE-C occupe 0.80 $nm^2$.
Dans cet exemple, on considère également qu'un groupe COOH réagit avec la fonction $NH_2$ du phospholipide pour former une liaison amide et que le rendement de la réaction de couplage est de 100%.

On suppose par ailleurs une distribution homogène des groupements COOH et un pourcentage de groupements COOH à la surface des SAM identique à celui des solutions utilisées pour la préparation des supports.
Si la surface réelle occupée par le groupement -COOH est inférieure à 0.80 $nm^2$, on divise la capacité de la SAM par 2.
1000 RU équivaut à 1 ng de phospholipide/$mm^2$
PM DOPE-C : 879g/mol

Les résultats obtenus sont présentés dans le tableau suivant.

| Support | Densité théorique de pilier DOPE-C | | Densité expérimentale de pilier DOPE-C | | Rendement (%) |
|---|---|---|---|---|---|
| | (RU) | (pmol /mm2) | (RU) | (pmol /mm2) | |
| Sia-100 | 1820 | 2.07 | 1956 | 2.22 | 107 |
| Sia- 70 | 1274 | 1.45 | 796 | 0.91 | 62 |
| Sia-33 | 1208 | 1.37 | 394 | 0.45 | 33 |
| Sia-20 | 728 | 0.83 | 512 | 0.58 | 70 |
| Sia-11 | 400 | 0.45 | 417 | 0.47 | 104 |
| * Valeur moyenne calculée sur la base de 2 expériences | | | | | |

En considérant que le rendement de réaction de couplage peut varier de 70 à 100% mais également des approximations dans le calcul théorique, la densité expérimentale de pilier DOPE-C est proche de la valeur théorique (mis à part la valeur obtenue pour Sia-33 qui est faible)

<u>Stabilité</u>

Le tableau suivant indique les variations de RU obtenues avant et après chaque injection :

| Support | Membrane de POPC | NaOH 20mM 100µl/min | NaOH 100 mM 10µl/min | HCl 100 mM 10µl/ min | Surface |
|---|---|---|---|---|---|
| sia-100 | 866 | -150 | -55 | -55 | fc1=0 (sans pilier) |
| sia-100 | 799 | -92 | -39 | 40 | fc4=1956 RU (avec pilier) |
| sia-70 | 961 | -139 | -33 | -28 | fc1=0 (sans pilier) |
| sia-70 | 1271 | -129 | -33 | 3 | fc2=951 RU (avec pilier) |
| sia-70 | 1641 | -107 | -45 | 71 | fc3=504 RU (avec pilier) |
| sia-70 | 1127 | -79 | -43 | 28 | fc4=642 RU (avec pilier) |
| sia-33 | 906 | -248 | -46 | -28 | fc1=0(sans pilier) |
| sia-33 | 1263 | -114 | -42 | 3 | fc2=402 RU (avec pilier) |
| sia-33 | 1979 | -171 | -57 | 138 | fc3=386 RU (avec pilier) |
| sia-33 | 1041 | -107 | -54 | 87 | fc4=0 (sans pilier) |
| sia-0 | 1653 | -155 | -48 | -4 | fc1=0 (sans pilier) |
| sia-0 | 1157 | -108 | -55 | 125 | fc4=0 (sans pilier) |

Les stabilités des membranes supportées formées à partir du lipide POPC sont semblables pour l'ensemble des surfaces testées dans les conditions de l'expérience, i.e : Injection prolongée de tampon de course HBS-N, injection de NaOH 20 mM (100 µlmin, 1min), de NaOH et de HCl 100 mM (10µl/min10ul, 1 min).
Au niveau d'un même support, lorsque la surface testée ne porte pas de pilier la stabilité est moindre que les surfaces portant des piliers.

## Revendications

1. Méthode de préparation d'une membrane artificielle supportée comprenant les étapes suivantes :

   a) mettre une surface fonctionnalisée en présence d'un mélange de ligands spécifiques (L) et de piliers phospholipidiques (P) susceptibles de se lier à ladite surface fonctionnalisée, et
   b) mettre la surface obtenue en a) en présence de lipides de manière à permettre la formation d'une bicouche lipidique supportée par les piliers phospholipidiques (P).

2. Méthode selon la revendication 1, **caractérisée en ce que** la surface fonctionnalisée comporte des molécules porteuses de groupements fonctionnels permettant de lier, de manière covalente, les piliers phospholipidiques (P) et/ou les ligands spécifiques (L), et des molécules porteuses de groupements non fonctionnels.

3. Méthode selon la revendication 2, **caractérisée en ce que** la surface fonctionnalisée comporte des molécules porteuses de groupements fonctionnels liant spécifiquement les piliers phospholipidiques (P) et d'autres molécules porteuses de groupements fonctionnels liant spécifiquement les ligands spécifiques (L).

4. Méthode selon la revendication 2, **caractérisée en ce que** la surface fonctionnalisée comporte des molécules porteuses de groupements fonctionnels X, éventuellement activables, liant les ligands spécifiques (L) et les piliers phospholipidiques (P), et des molécules porteuses de groupements non fonctionnels Z.

5. Méthode selon la revendication 3, **caractérisée en ce que** la surface fonctionnalisée comporte un mélange de molécules porteuses de groupements fonctionnels X et Y, les groupements X et Y liant sélectivement les ligands spécifiques (L) et les piliers (P), respectivement.

6. Méthode selon l'une des revendications 4 ou 5, **caractérisée en ce que** les molécules porteuses de groupements fonctionnels ou non fonctionnels sont des dérivés thiols de formule $HS-(CH_2)n-X$, $HS-(CH_2)m-Y$ ou $HS-(CH_2)n-Z$ dans lesquelles n et m, identiques ou différents, représentent un nombre entier compris entre 2 et 15.

7. Méthode de préparation d'une membrane supportée, comprenant les étapes suivantes :

   a) recouvrir une surface par des molécules de formule $HS-(CH_2)n-X$ et $HS-(CH_2)m-Z$, X étant un groupement fonctionnel préalablement activé capable d'immobiliser un ligand spécifique (L) ou un pilier phospholipidique (P), Z étant un groupement non-fonctionnel incapable de réagir avec ledit ligand (L) ou pilier (P) et n et m étant compris entre 2 et 15,
   b) mettre la surface fonctionnalisée obtenue en a) en présence d'un mélange contenant les ligands spécifiques (L) et les piliers phospholipidiques (P), puis
   c) mettre la surface obtenue en b) en présence de lipides de manière à permettre la formation d'une bicouche lipidique supportée par les piliers phospholipidiques (P).

8. Méthode de préparation d'une membrane supportée, comprenant les étapes suivantes :

   a) recouvrir une surface par un mélange de molécules de formule $HS-(CH_2)n-X$, $HS-(CH_2)m-Y$ et $HS-(CH_2)m-Z$, X et Y étant des groupements fonctionnels préalablement activés capables d'immobiliser respectivement un pilier phospholipidique (P) et un ligand spécifique (L), Z étant un groupement non-fonctionnel incapable de réagir avec ledit ligand (L) ou pilier (P) et n et m étant compris entre 2 et 15,
   b) mettre la surface fonctionnalisée obtenue en a) en présence d'un mélange contenant les ligands spécifiques (L) et les piliers phospholipidiques (P), puis
   c) mettre la surface obtenue en b) en présence de lipides de manière à permettre la formation d'une bicouche lipidique supportée par les piliers phospholipidiques (P).

9. Méthode selon la revendication 7 ou 8, **caractérisée en ce que**, dans l'étape b), on met la surface obtenue en contact avec les piliers phospholipidiques (P) dans un premier temps puis, dans un second temps, avec les ligands spécifiques (L).

10. Méthode selon l'une des revendications 7 à 9, **caractérisée en ce qu'**elle comprend une étape supplémentaire, suivant l'étape a), de désactivation des groupements X ou Y n'ayant pas réagi avec les ligands (L) ou les piliers (P).

**11.** Méthode selon l'une quelconque des revendications précédentes, **caractérisée en ce qu'**elle comprend une étape supplémentaire d'introduction d'une ou plusieurs protéines dans la membrane supportée.

**12.** Méthode selon l'une quelconque des revendications précédentes, **caractérisée en ce que** les lipides utilisés dans l'étape c) sont des extraits cellulaires, des fragments membranaires, des micelles, des monocouches ou des bicouches lipidiques, des vésicules lipidiques et/ou des lipides, phospholipides, lipopeptides ou lipides biotinylés, seuls ou en mélanges.

**13.** Méthode selon l'une quelconque des revendications précédentes, **caractérisée en ce que** la surface utilisée est une surface en or, verre, diamant, silicium, dioxyde de silicium ($S_iO_2$), nitrite de silicone, oxyde de tantale ($Ta_2O_5$), dioxyde de titane($T_iO_2$), nitrite de titane, carbide de titane, platine, tungstène, aluminium ou oxyde d'indium/étain, ou à base de ces matériaux.

**14.** Méthode selon la revendication 13, **caractérisée en ce que** la surface est une surface métallique, de préférence à base ou en or.

**15.** Méthode selon l'une quelconque des revendications 6 à 14, **caractérisée en ce que** les nombres n et m sont égaux à 10 ou 11.

**16.** Méthode selon l'une des revendications 2 à 15, **caractérisée en ce que** les groupements fonctionnels X et Y sont choisis parmi les groupes COOH, CHO, OH, $NH_2$, maléimide et biotine.

**17.** Méthode selon l'une des revendications 2 à 16, **caractérisée en ce que** le rapport molaire des groupements fonctionnels X et Y sur les groupements non-fonctionnels est compris entre 0,05 et 20, de préférence entre 0,1 et 5 et de manière encore plus préférée entre 0,2 et 0,3.

**18.** Méthode selon l'une quelconque des revendications précédentes, **caractérisée en ce que** le ligand spécifique (L) comprend un groupement d'affinité spécifique pour une molécule d'intérêt, un point d'ancrage à la surface fonctionnalisée et un espaceur, dont la longueur est comprise préférentiellement entre 3 et 500 Å.

**19.** Méthode selon la revendication 18, **caractérisée en ce que** le ligand (L) comporte un groupement d'affinité spécifique pour une molécule d'intérêt choisie parmi une protéine, un substrat, un antigène, un haptène, une lectine, un biorécepteur, un oligonucléotide ou une immunoglobuline ou une région de ceux-ci, de préférence une protéine.

**20.** Méthode selon la revendication 19, **caractérisée en ce que** le groupement d'affinité spécifique du ligand (L) est un anticorps ou un fragment d'anticorps spécifique d'une protéine membranaire.

**21.** Méthode selon l'une quelconque des revendications précédentes, **caractérisée en ce que** le pilier phospholipidique (P) comprend un point d'ancrage à la surface, un espaceur et un phospholipide.

**22.** Méthode selon la revendication 21, **caractérisée en ce que** le pilier phospholipidique (P) comprend un espaceur dont la longueur est inférieure à 500 Å, de préférence comprise entre 3 et 500 Å.

**23.** Membrane artificielle supportée **caractérisée en ce qu'**elle comprend une bicouche lipidique reliée à un support par des piliers phospholipidiques (P) ménageant un espace entre la bicouche et le support, et **en ce qu'**elle comprend un ou plusieurs ligands (L) spécifiques d'une molécule d'intérêt reliés de manière covalente au support et exposés dans ledit espace.

**24.** Membrane artificielle supportée **caractérisée en ce qu'**elle est susceptible d'être obtenue par une méthode selon l'une des revendications 1 à 22.

**25.** Utilisation d'une membrane supportée selon la revendication 23 ou 24, ou obtenue par une méthode selon l'une quelconque des revendications 1 à 22, pour la purification de protéines membranaires.

**26.** Utilisation d'une membrane supportée selon la revendication 23 ou 24, ou obtenue par une méthode selon l'une quelconque des revendications 1 à 22, pour la capture réversible de protéines membranaires ou de complexes protéiques membranaires.

**27.** Utilisation d'une membrane supportée selon la revendication 23 ou 24, ou obtenue par une méthode selon l'une quelconque des revendications 1 à 22, pour le criblage de composés interagissant avec des protéines membranaires.

**28.** Utilisation d'une membrane supportée selon la revendication 23 ou 24, ou obtenue par une méthode selon l'une quelconque des revendications 1 à 22, pour l'analyse des interactions protéine-protéine au sein d'un membrane.

**29.** Procédé de purification d'une protéine membranaire, comprenant la mise en présence d'une membrane supportée selon la revendication 23 ou 24, ou obtenue par une méthode selon l'une quelconque des revendications 1 à 22, avec une préparation membranaire comprenant la protéine à purifier, dans des conditions permettant l'introduction de ladite protéine dans ladite membrane supportée et l'application d'un flux lipidique.

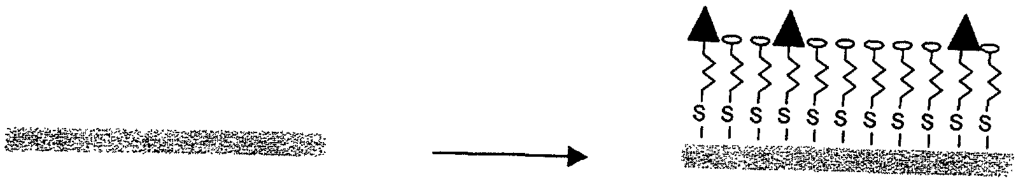

Thiol derivatives

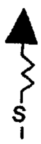 HS-$(CH_2)_{10}$-COOH

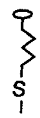 HS-$(CH_2)_{11}$-OH

| Support | | HS-$(CH_2)_{10}$-COOH (mol %) |
|---|---|---|
| SIA-$C_{11}$OOH | Sia-100 | 100 |
| SIA-$C_{11}$OOH / $C_{11}$OH-7/3 | Sia-70 | 70 |
| SIA-$C_{11}$OOH / $C_{11}$OH-1/2 | Sia-33 | 33 |
| SIA-$C_{11}$OOH / $C_{11}$OH-1/4 | Sia-20 | 20 |
| SIA-$C_{11}$OOH / $C_{11}$OH-1/8 | Sia-11 | 11 |
| SIA-$C_{11}$OH | Sia-0 | 0 |

FIGURE 1

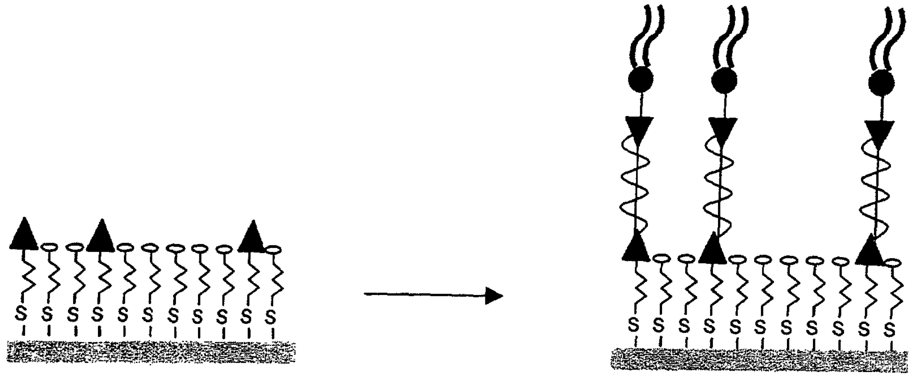

piliers phopholipides 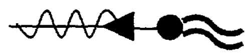

PE (R1 16:0 R2 18:1)

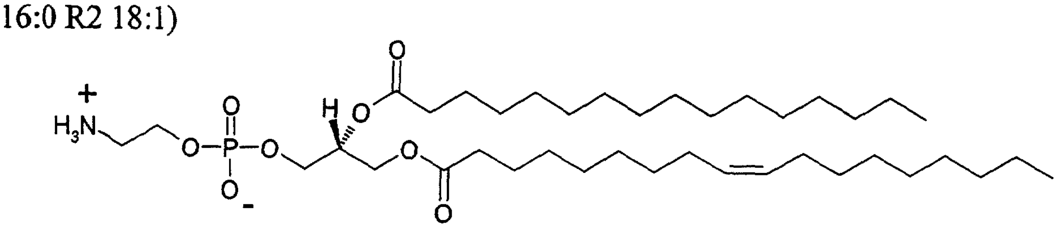

DOPE-C (R1=R2 18:1)

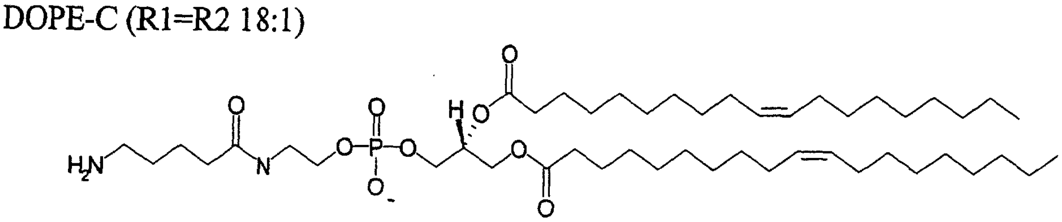

DPPE-C (R1=R2 16:0)

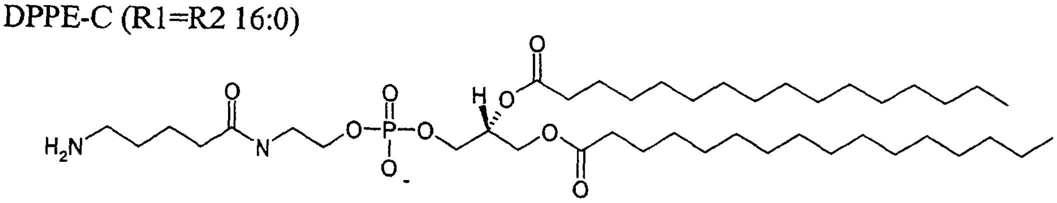

DPPE-PEG (R1=R2 16:0 )

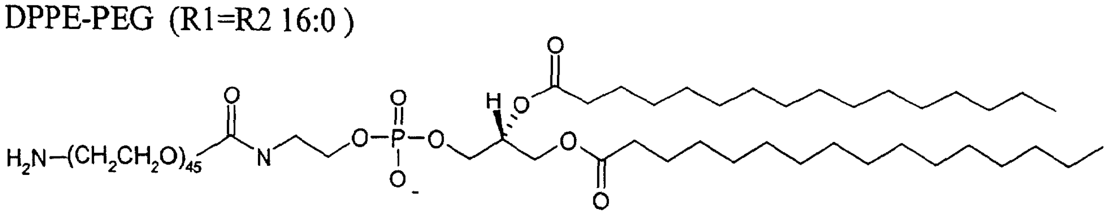

**FIGURE 2**

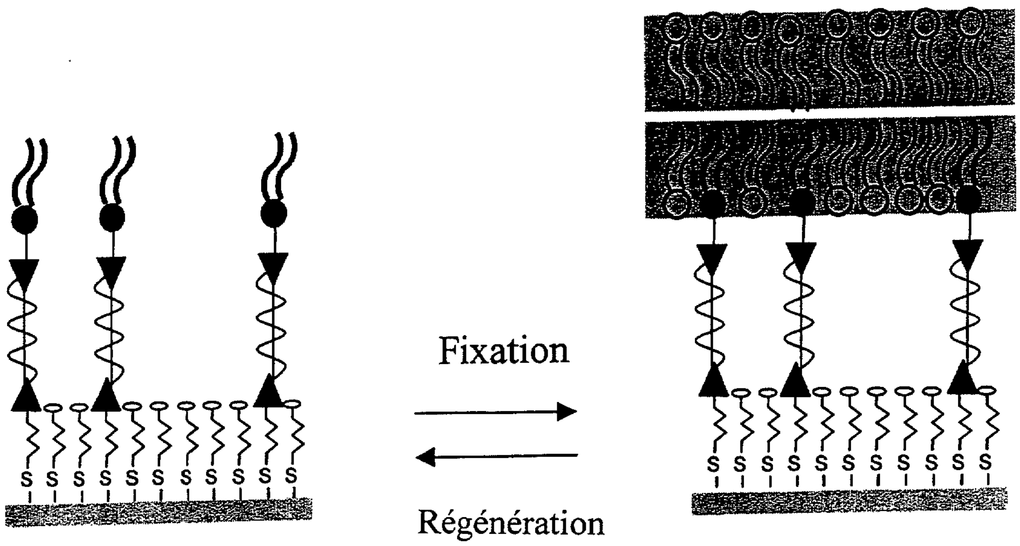


POPC ($R_1$ 16:0 $R_2$ 18:1) :

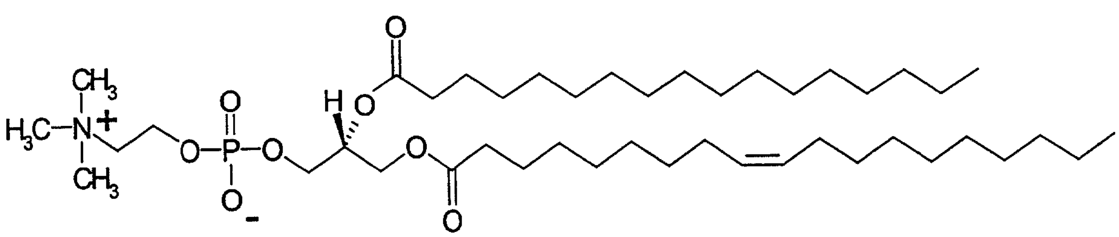

FIGURE 3

# FIGURE 4

| Support, | BSA (2) | | | Micelles -4 | | NaOH (5) et (6) | | | BSA (7) | | | BSA (9) | | | n | Canal |
|---|---|---|---|---|---|---|---|---|---|---|---|---|---|---|---|---|
| | I | F | Δ F-I | I | F | I | F | Δ F-I | I | F | Δ F-I | I | F | Δ F-I | | |
| sia-100 | -79 | 192 | 271 | 0 | 672 | 818 | 588 | -230 | 622 | 789 | 167 | -13 | 203 | 216 | 1 | fc3=0 (sans pilier) |
| | -46 | 54 | 100 | 0 | 424 | 759 | 694 | -65 | 728 | 608 | -120 | -325 | -238 | 87 | | fc4=1956 |
| sia-100 | -61 | 119 | 180 | 0 | 705 | 829 | 659 | -170 | 675 | 798 | 123 | -28 | 158 | 186 | 2 | fc3=0 (sans pilier) |
| | -50 | 51 | 101 | 0 | 717 | 1028 | 965 | -63 | 991 | 893 | -98 | -45 | 80 | 125 | | fc4=1956 |
| sia-100 | -60 | 163 | 223 | 0 | 790 | 918 | 656 | -262 | 673 | 905 | 232 | -172 | 35 | 207 | 3 | fc3=0 (sans pilier) |
| | -41 | 235 | 276 | 0 | 638 | 904 | 766 | -138 | 797 | 806 | 9 | -420 | -114 | 306 | | fc4=1956 |
| sia-100 | -43 | 129 | 172 | 0 | 969 | 1091 | 876 | -215 | 891 | 1064 | 173 | -33 | 127 | 160 | 4 | fc3=0 (sans pilier) |
| | -45 | 227 | 272 | 0 | 1032 | 1279 | 1122 | -157 | 1147 | 1159 | 12 | -38 | 240 | 278 | | fc4=1956 |
| sia-70 | -85 | -31 | 54 | 0 | 580 | 721 | 481 | -240 | 506 | 494 | -12 | -6 | 8 | 14 | 1 | fc1=0 (sans pilier) |
| | -50 | 112 | 162 | 0 | 1059 | 1286 | 1139 | -147 | 1185 | 1096 | -89 | 44 | 200 | 156 | | fc2=951 |
| | -67 | 23 | 90 | 0 | 1590 | 1821 | 1662 | -159 | 1710 | 1579 | -131 | -34 | 61 | 95 | | fc3=504 |
| | -44 | 37 | 81 | 0 | 1136 | 1304 | 1174 | -130 | 1221 | 1166 | -55 | 90 | 177 | 87 | | fc4=642 |
| sia-70 | -134 | 146 | 280 | 0 | 547 | 738 | 520 | -218 | 546 | 681 | 135 | -133 | 55 | 188 | 2 | fc1=0 (sans pilier) |
| | -130 | 317 | 447 | 0 | 845 | 917 | 837 | -80 | 862 | 923 | 61 | -162 | 169 | 331 | | fc2=951 |
| | -119 | 300 | 419 | 0 | 1757 | 1850 | 1746 | -104 | 1774 | 1800 | 26 | -85 | 437 | 522 | | fc3=504 |
| | -75 | 261 | 336 | 0 | 1152 | 1230 | 1145 | -85 | 1177 | 1249 | 72 | -119 | 256 | 375 | | fc4=642 |
| sia-70 | | | | 0 | 643 | 773 | 525 | -248 | 539 | 531 | -8 | | | | 3 | fc1=0 (sans pilier) |
| | | | | 0 | 1370 | 1553 | 1441 | -112 | 1469 | 1404 | -65 | | | | | fc2=951 |
| | | | | 0 | 1381 | 1574 | 1418 | -156 | 1435 | 1393 | -42 | | | | | fc3=504 |
| | | | | 0 | 1279 | 1480 | 1341 | -139 | 1368 | 1315 | -53 | | | | | fc4=642 |
| sia-70 | | | | 0 | 821 | 913 | 797 | -116 | 806 | 798 | -8 | | | | 4 | fc1=0 (sans pilier) |
| | | | | 0 | 1606 | 1730 | 1687 | -43 | 1685 | 1666 | -19 | | | | | fc2=951 |
| | | | | 0 | 1140 | 1231 | 1178 | -53 | 1182 | 1186 | 4 | | | | | fc3=504 |
| | | | | 0 | 1130 | 1228 | 1168 | -60 | 1165 | 1167 | 2 | | | | | fc4=642 |
| sia33 | -26 | -20 | 6 | 0 | 503 | 661 | 542 | -119 | 568 | 472 | -96 | -124 | -102 | 22 | 1 | fc1=0 (sans pilier) |
| | -50 | -29 | 21 | 0 | 670 | 852 | 763 | -89 | 798 | 699 | -99 | -103 | -81 | 22 | | fc2=402 |
| | -39 | 16 | 55 | 0 | 1462 | 1674 | 1590 | -84 | 1621 | 1505 | -116 | -200 | -132 | 68 | | fc3=386 |
| | -64 | 46 | 110 | 0 | 689 | 876 | 767 | -109 | 810 | 740 | -70 | -201 | -85 | 116 | | fc4=0 |

FIGURE 4

| | | | | | | | | | | | | | | | | |
|---|---|---|---|---|---|---|---|---|---|---|---|---|---|---|---|---|
| sia33 | -35 | 26 | 61 | 0 | 698 | 855 | 695 | -160 | 722 | 663 | -59 | -146 | -71 | 75 | 2 | fc1=0 (sans pilier) |
| | -52 | 50 | 102 | 0 | 855 | 1034 | 901 | -133 | 941 | 894 | -47 | -58 | 41 | 99 | | fc2=402 |
| | -41 | 77 | 118 | 0 | 1384 | 1599 | 1467 | -132 | 1516 | 1429 | -87 | -205 | -74 | 131 | | fc3=386 |
| | -60 | 83 | 143 | 0 | 497 | 690 | 549 | -141 | 606 | 571 | -35 | -273 | -136 | 137 | | fc4=0 |
| sia33 | -41 | 451 | 492 | 0 | 504 | 637 | 533 | -104 | 560 | 537 | -13 | -37 | 32 | 69 | 3 | fc1=0 (sans pilier) |
| | -43 | -115 | -72 | 0 | 829 | 976 | 843 | -133 | 869 | 863 | -6 | -16 | 77 | 93 | | fc2=402 |
| | -45 | -262 | -217 | 0 | 1166 | 1323 | 1194 | -129 | 1225 | 1202 | -23 | -52 | 68 | 120 | | fc3=386 |
| | -61 | 230 | 291 | 0 | 662 | 808 | 682 | -126 | 722 | 713 | -9 | -97 | 20 | 117 | | fc4=0 |
| sia33 | | | | 0 | 681 | 828 | 687 | -141 | 724 | 679 | -45 | | | | 4 | fc1=0 (sans pilier) |
| | | | | 0 | 1209 | 1363 | 1241 | -122 | 1280 | 1213 | -67 | | | | | fc2=402 |
| | | | | 0 | 1336 | 1473 | 1365 | -108 | 1404 | 1362 | -42 | | | | | fc3=386 |
| | | | | 0 | 797 | 952 | 846 | -106 | 875 | 849 | -26 | | | | | fc4=0 |
| sia-20 | -35 | 144 | 179 | 0 | 653 | 795 | 655 | -140 | 675 | 749 | 74 | -23 | 159 | 182 | 1 | fc1=0 (sans pilier) |
| | -33 | 108 | 141 | 0 | 1031 | 1220 | 1130 | -90 | 1158 | 1163 | 5 | 15 | 152 | 137 | | fc2=494 |
| | -1 | 96 | 97 | 0 | 1602 | 1798 | 1702 | -96 | 1726 | 1668 | -58 | -2 | 96 | 98 | | fc3=531 |
| | -40 | -39 | 1 | 0 | 393 | 541 | 422 | -119 | 470 | 379 | -91 | 30 | -18 | -48 | | fc4=0 (sans pilier) |
| sia-20 | -84 | 157 | 241 | 0 | 431 | 593 | 470 | -123 | 483 | 588 | 105 | -105 | 92 | 197 | 2 | fc1=0 (sans pilier) |
| | -84 | 219 | 303 | 0 | 1031 | 1256 | 1123 | -133 | 1153 | 1219 | 66 | -74 | 188 | 262 | | fc2=494 |
| | -87 | 201 | 288 | 0 | 1354 | 1554 | 1424 | -130 | 1457 | 1497 | 40 | -80 | 172 | 252 | | fc3=531 |
| | -81 | 136 | 217 | 0 | 406 | 557 | 438 | -119 | 478 | 584 | 106 | -78 | 111 | 189 | | fc4=0 (sans pilier) |
| sia-20 | -158 | 201 | 359 | 0 | 419 | 627 | 458 | -169 | 481 | 658 | 177 | -384 | -113 | 271 | 3 | fc1=0 (sans pilier) |
| | -82 | 279 | 361 | 0 | 713 | 970 | 833 | -137 | 869 | 1003 | 134 | -313 | 13 | 326 | | fc2=494 |
| | -71 | 276 | 347 | 0 | 1227 | 1483 | 1351 | -132 | 1386 | 1446 | 60 | -298 | 11 | 309 | | fc3=531 |
| | -152 | 183 | 335 | 0 | 375 | 579 | 428 | -151 | 472 | 622 | 150 | -316 | -62 | 254 | | fc4=0 (sans pilier) |
| sia-20 | | | | 0 | 432 | 592 | 447 | -145 | 476 | 489 | 13 | | | | 4 | fc1=0 (sans pilier) |
| | | | | 0 | 479 | 638 | 522 | -116 | 560 | 613 | 53 | | | | | fc2=494 |
| | | | | 0 | 1093 | 1259 | 1163 | -96 | 1197 | 1196 | -1 | | | | | fc3=531 |
| | | | | 0 | 418 | 558 | 478 | -80 | 504 | 529 | 25 | | | | | fc4=0 (sans pilier) |
| sia-11 | -108 | 88 | 196 | 0 | 705 | 893 | 754 | -139 | 778 | 836 | 63 | -46 | 102 | 148 | 1 | fc1=0 (sans pilier) |
| | -104 | 83 | 187 | 0 | 866 | 1128 | 1009 | -119 | 1037 | 1055 | 18 | -71 | 86 | 157 | | fc2=0 (sans pilier) |
| | -123 | -7 | 116 | 0 | 1329 | 1603 | 1478 | -125 | 1511 | 1451 | -60 | -102 | -11 | 91 | | fc3=386 |
| | -121 | -66 | 55 | 0 | 1040 | 1280 | 1159 | -121 | 1208 | 1058 | -150 | -37 | -55 | -18 | | fc4=449 |
| sia-11 | -56 | 32 | 88 | 0 | 772 | 983 | 814 | -169 | 862 | 794 | -58 | -350 | -272 | 78 | 2 | fc1=0 (sans pilier) |
| | -79 | 73 | 152 | 0 | 627 | 894 | 763 | -131 | 809 | 770 | -39 | -289 | -146 | 143 | | fc2=0 (sans pilier) |
| | -85 | 53 | 138 | 0 | 969 | 1273 | 1146 | -127 | 1193 | 1108 | -85 | -303 | -175 | 128 | | fc3=386 |
| | -67 | 115 | 182 | 0 | 727 | 972 | 847 | -125 | 897 | 862 | -35 | -356 | -189 | 167 | | fc4=449 |

FIGURE 4

| | | | | | | | | | | | | | | | | |
|---|---|---|---|---|---|---|---|---|---|---|---|---|---|---|---|---|
| sia-11 | -53 | 58 | 111 | 0 | 906 | 1081 | 958 | -123 | 980 | 974 | -7 | -48 | 54 | 102 | 3 | fc1=0 (sans pilier) |
| | -80 | 82 | 162 | 0 | 802 | 1027 | 911 | -116 | 940 | 930 | -10 | -53 | 82 | 135 | | fc2=0 (sans pilier) |
| | -82 | 56 | 138 | 0 | 1034 | 1281 | 1167 | -114 | 1198 | 1155 | -43 | -97 | 27 | 124 | | fc3=386 |
| | -86 | 90 | 176 | 0 | 932 | 1120 | 1009 | -111 | 1046 | 1045 | -1 | -121 | 33 | 154 | | fc4=449 |
| sia-11 | | | | 0 | 932 | 1122 | 1022 | -100 | 1044 | 993 | -51 | | | | 4 | fc1=0 (sans pilier) |
| | | | | 0 | 1065 | 1267 | 1179 | -88 | 1200 | 1167 | -33 | | | | | fc2=0 (sans pilier) |
| | | | | 0 | 1026 | 1221 | 1134 | -87 | 1146 | 1122 | -24 | | | | | fc3=386 |
| | | | | 0 | 1083 | 1305 | 1143 | -162 | 1161 | 1128 | -33 | | | | | fc4=449 |
| sia-0 | -98 | 17 | 115 | 0 | 916 | 1109 | 990 | -119 | 1040 | 980 | -60 | -280 | -166 | 114 | 1 | fc1=0 (sans pilier) |
| | -151 | -12 | 139 | 0 | 1160 | 1402 | 1237 | -165 | 1325 | 1252 | -73 | -82 | 36 | 118 | | fc4=0 (sans pilier) |
| sia-0 | -77 | 56 | 133 | 0 | 1048 | 1304 | 1199 | -105 | 1249 | 22 | -1227 | -107 | 22 | 129 | 2 | fc1=0 (sans pilier) |
| | -96 | 42 | 138 | 0 | 1140 | 1381 | 1269 | -112 | 1336 | -143 | -1479 | -265 | -143 | 122 | | fc4=0 (sans pilier) |

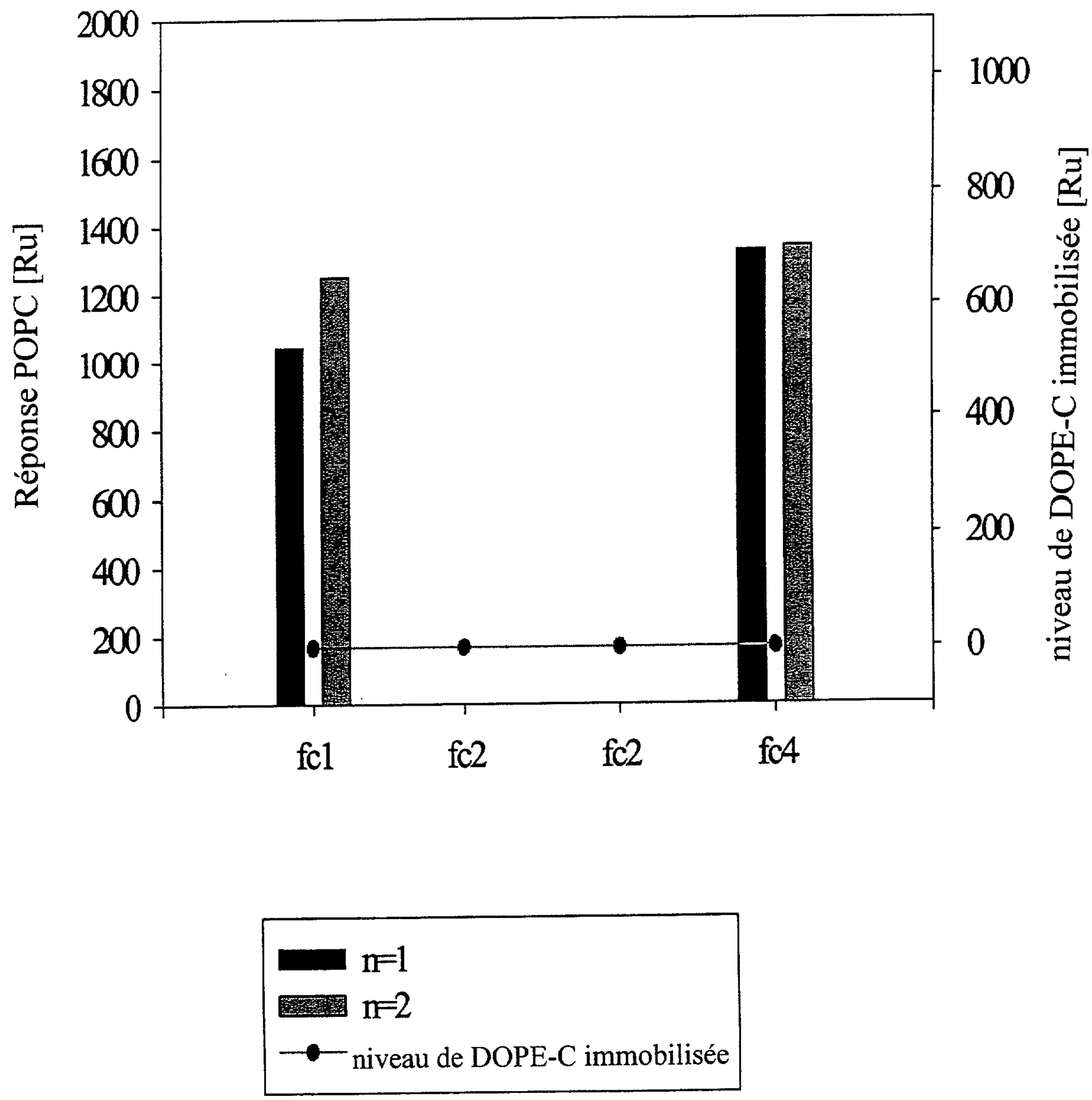
2000
1800
1600
1400
1200
1000
800
600
400
200
0
Réponse POPC [Ru]
1000
800
600
400
200
0
niveau de DOPE-C immobilisée [Ru]
fc1
fc2
fc2
fc4
n=1
n=2
niveau de DOPE-C immobilisée

**FIGURE 5a**

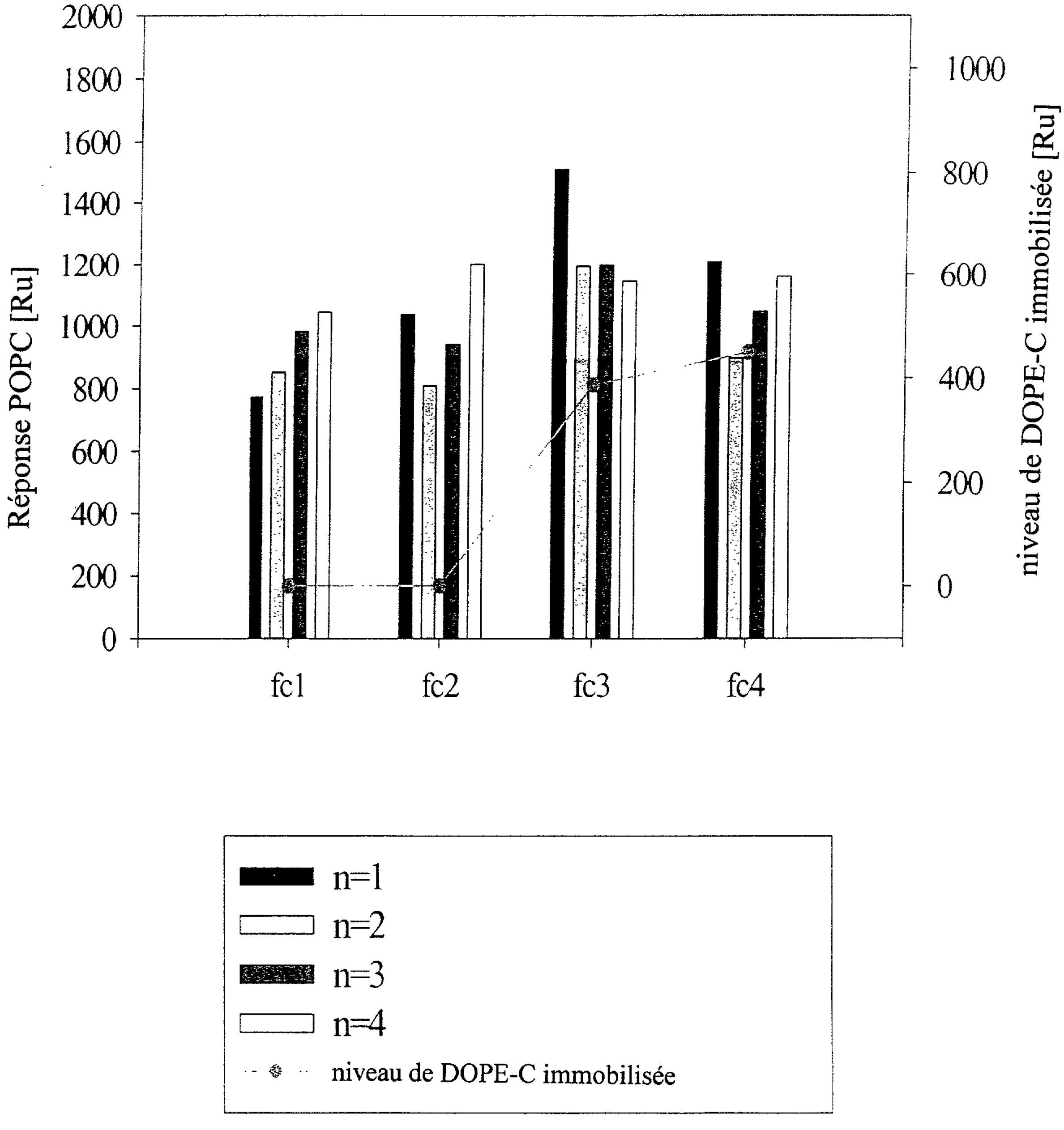
2000
1800
1600
1400
1200
1000
800
600
400
200
0
Réponse POPC [Ru]
1000
800
600
400
200
0
niveau de DOPE-C immobilisée [Ru]
fc1
fc2
fc3
fc4
n=1
n=2
n=3
n=4
niveau de DOPE-C immobilisée

FIGURE 5b

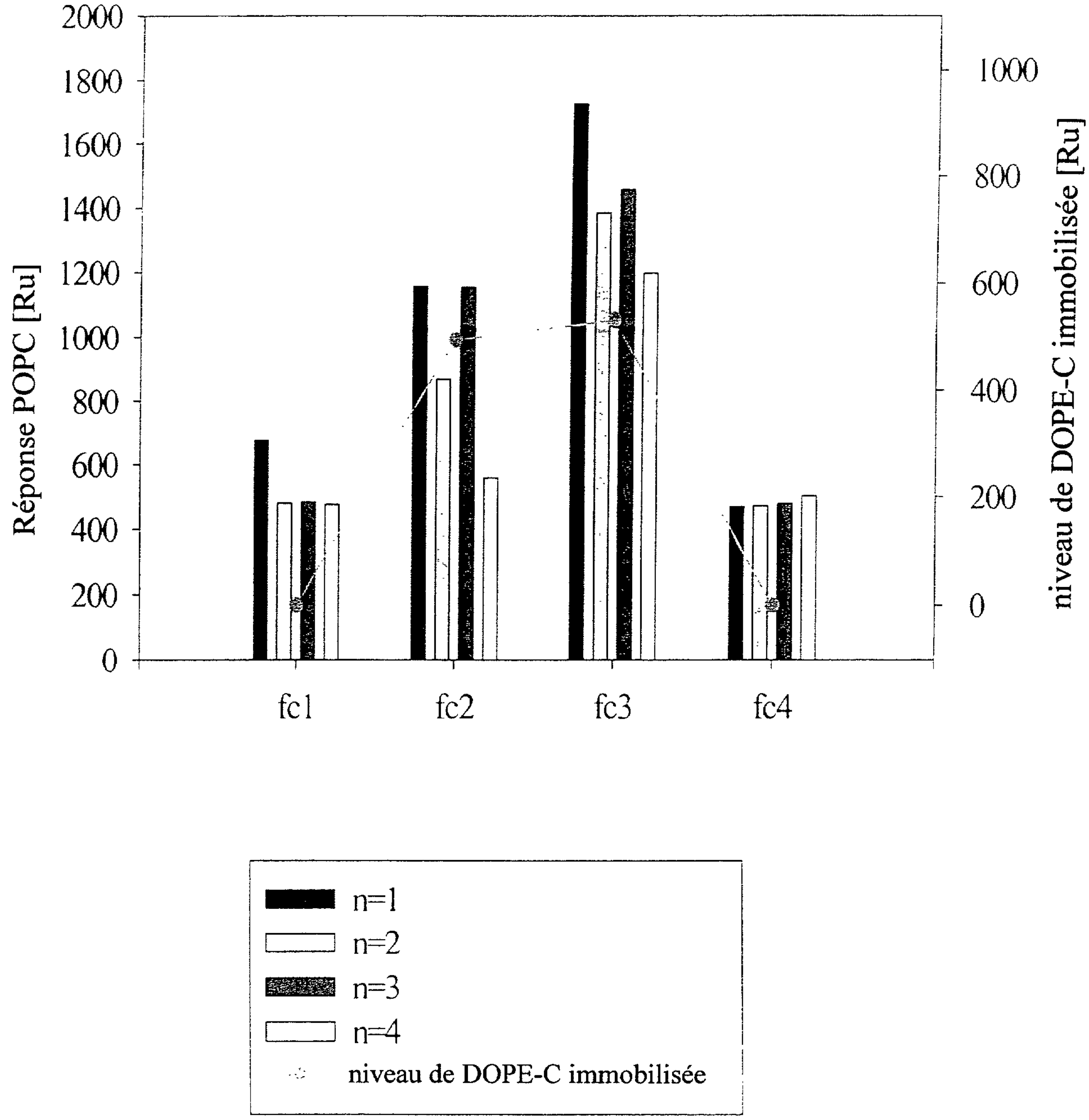
2000
1800
1600
1400
1200
1000
800
600
400
200
0
Réponse POPC [Ru]
1000
800
600
400
200
0
niveau de DOPE-C immobilisée [Ru]
fc1
fc2
fc3
fc4
n=1
n=2
n=3
n=4
niveau de DOPE-C immobilisée

FIGURE 5c

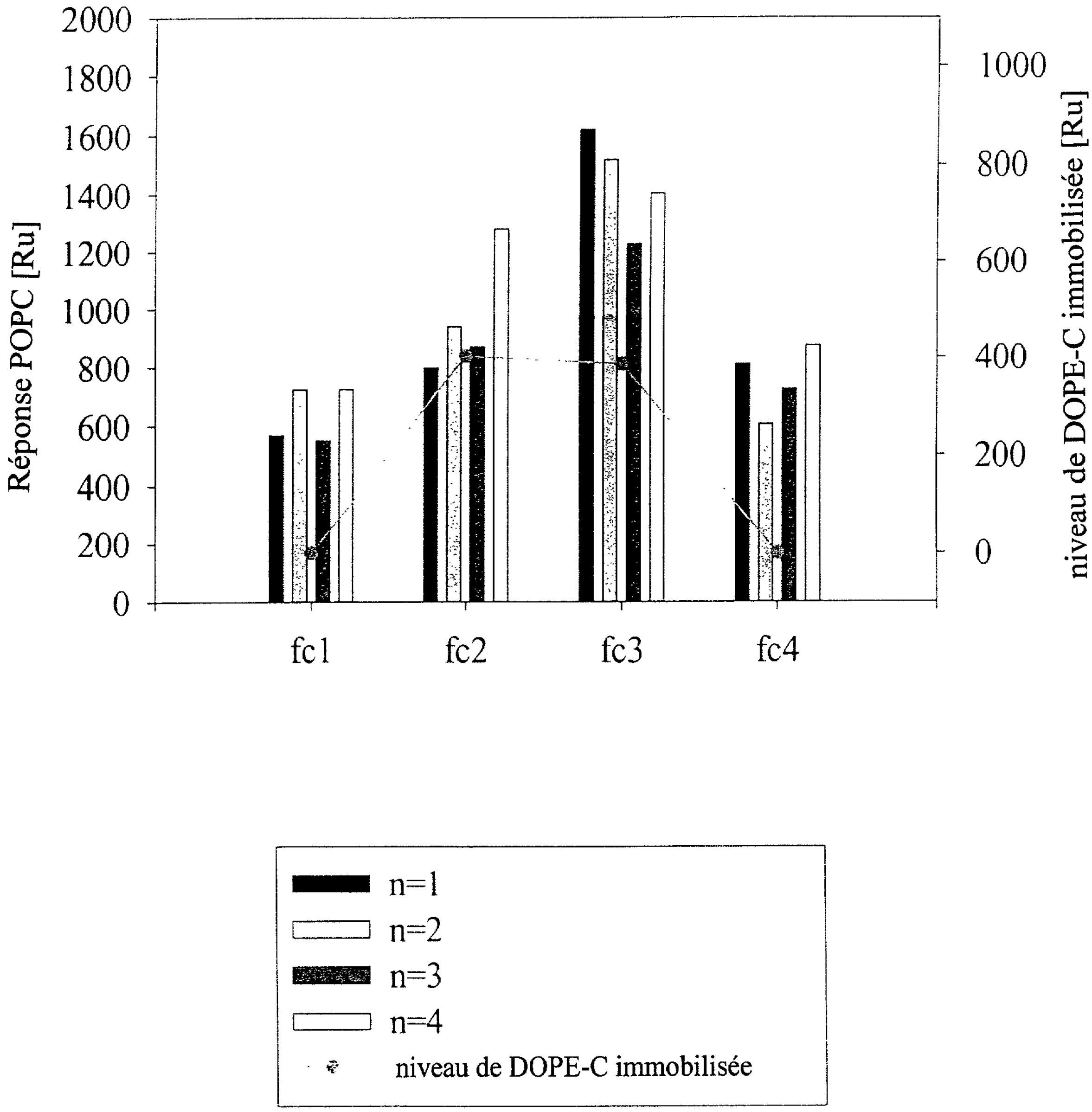
2000
1800
1600
1400
1200
1000
800
600
400
200
0
Réponse POPC [Ru]
1000
800
600
400
200
0
niveau de DOPE-C immobilisée [Ru]
fc1
fc2
fc3
fc4
n=1
n=2
n=3
n=4
niveau de DOPE-C immobilisée

FIGURE 5d

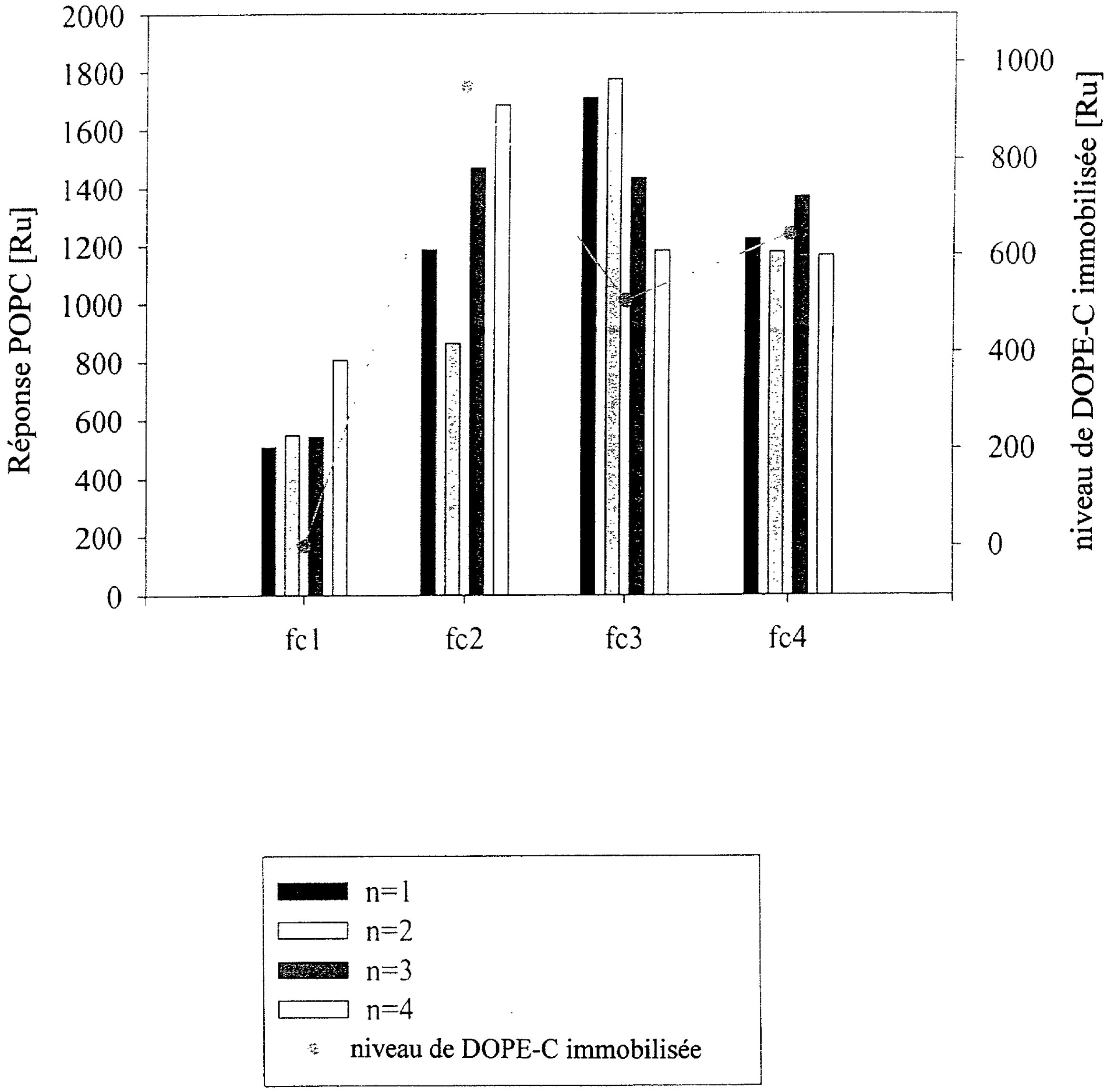
2000
1800
1600
1400
1200
1000
800
600
400
200
0
Réponse POPC [Ru]
1000
800
600
400
200
0
niveau de DOPE-C immobilisée [Ru]
fc1
fc2
fc3
fc4
n=1
n=2
n=3
n=4
niveau de DOPE-C immobilisée

FIGURE 5e

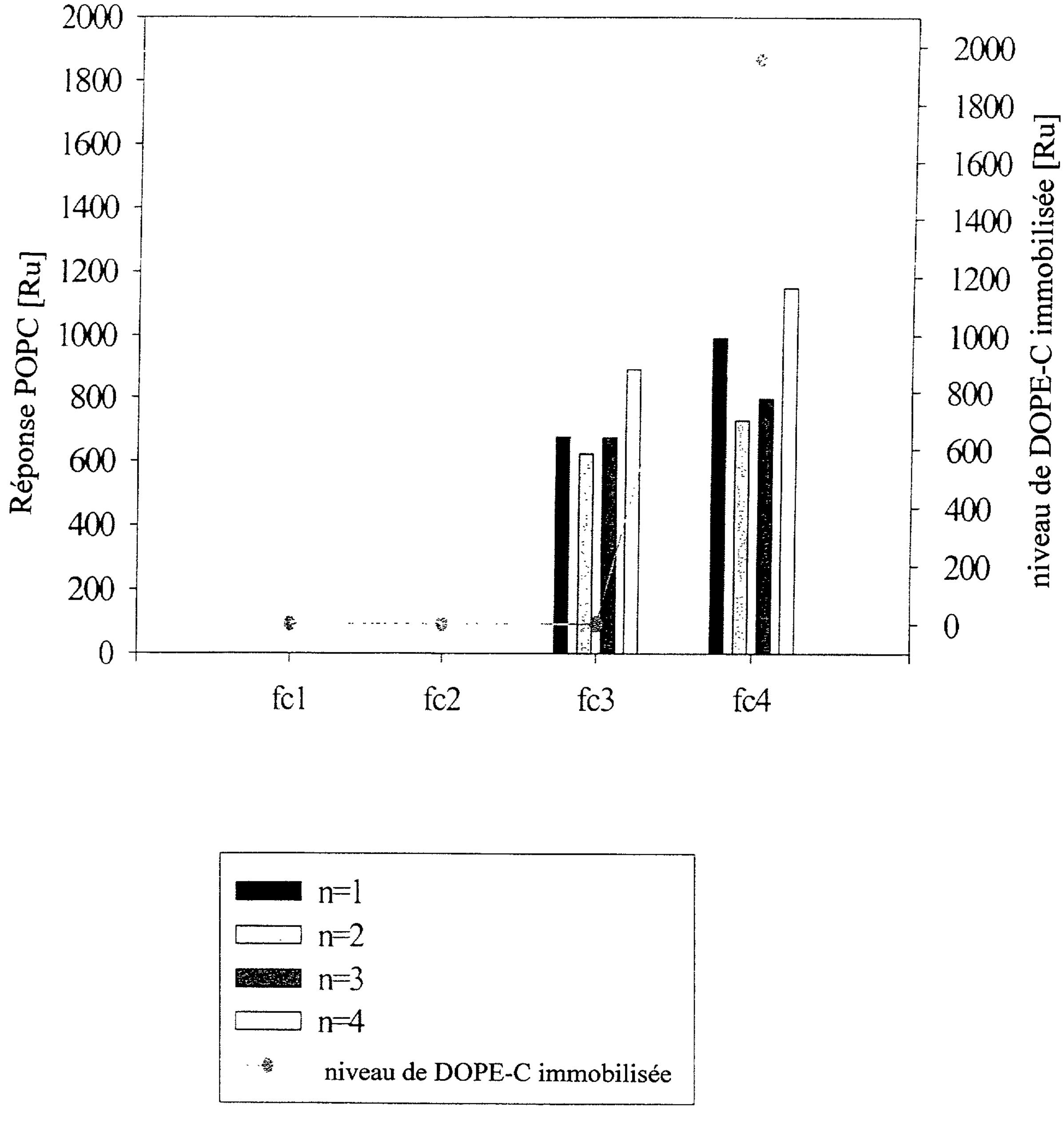
2000
1800
1600
1400
1200
1000
800
600
400
200
0
Réponse POPC [Ru]
2000
1800
1600
1400
1200
1000
800
600
400
200
0
niveau de DOPE-C immobilisée [Ru]
fc1
fc2
fc3
fc4
n=1
n=2
n=3
n=4
niveau de DOPE-C immobilisée

FIGURE 5f

Figure 6

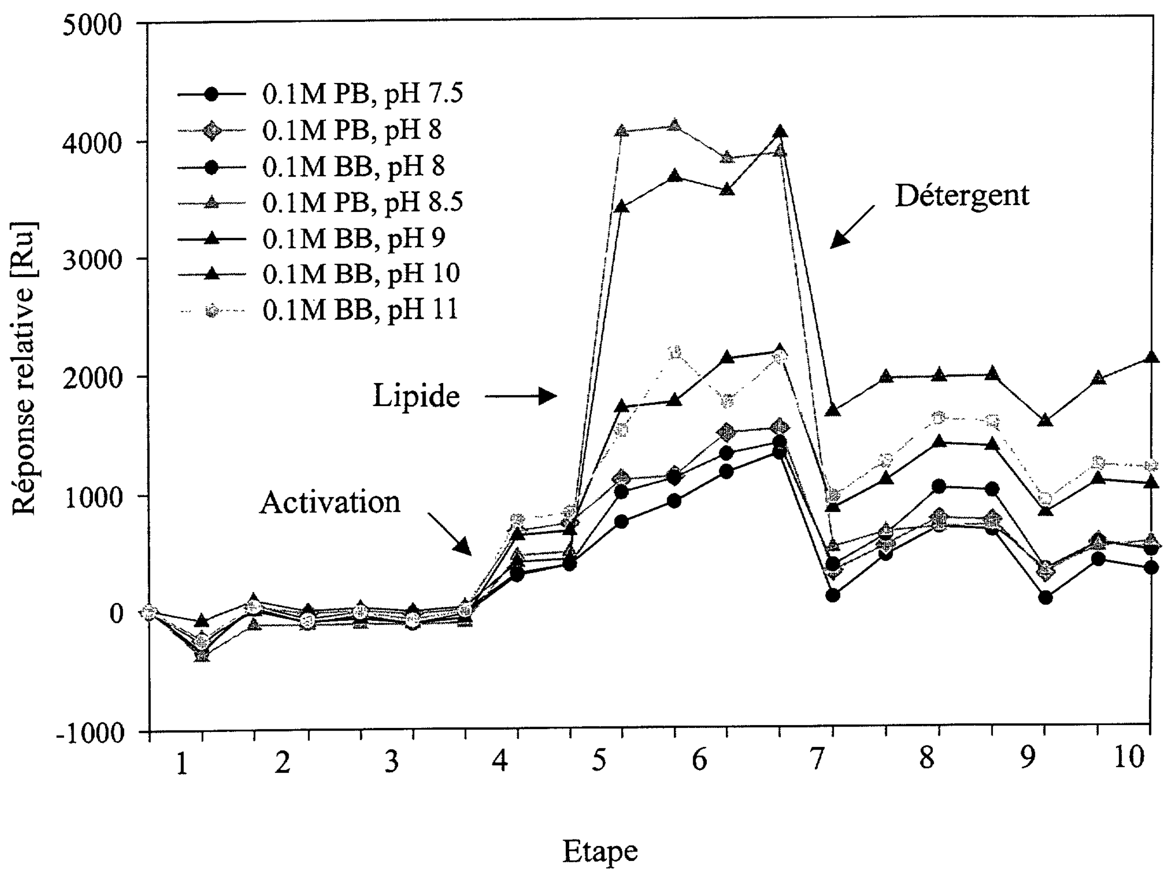
5000
4000
3000
2000
1000
0
-1000
Réponse relative [Ru]
0.1M PB, pH 7.5
0.1M PB, pH 8
0.1M BB, pH 8
0.1M PB, pH 8.5
0.1M BB, pH 9
0.1M BB, pH 10
0.1M BB, pH 11
Détergent
Lipide
Activation
1
2
3
4
5
6
7
8
9
10
Etape

Figure 7

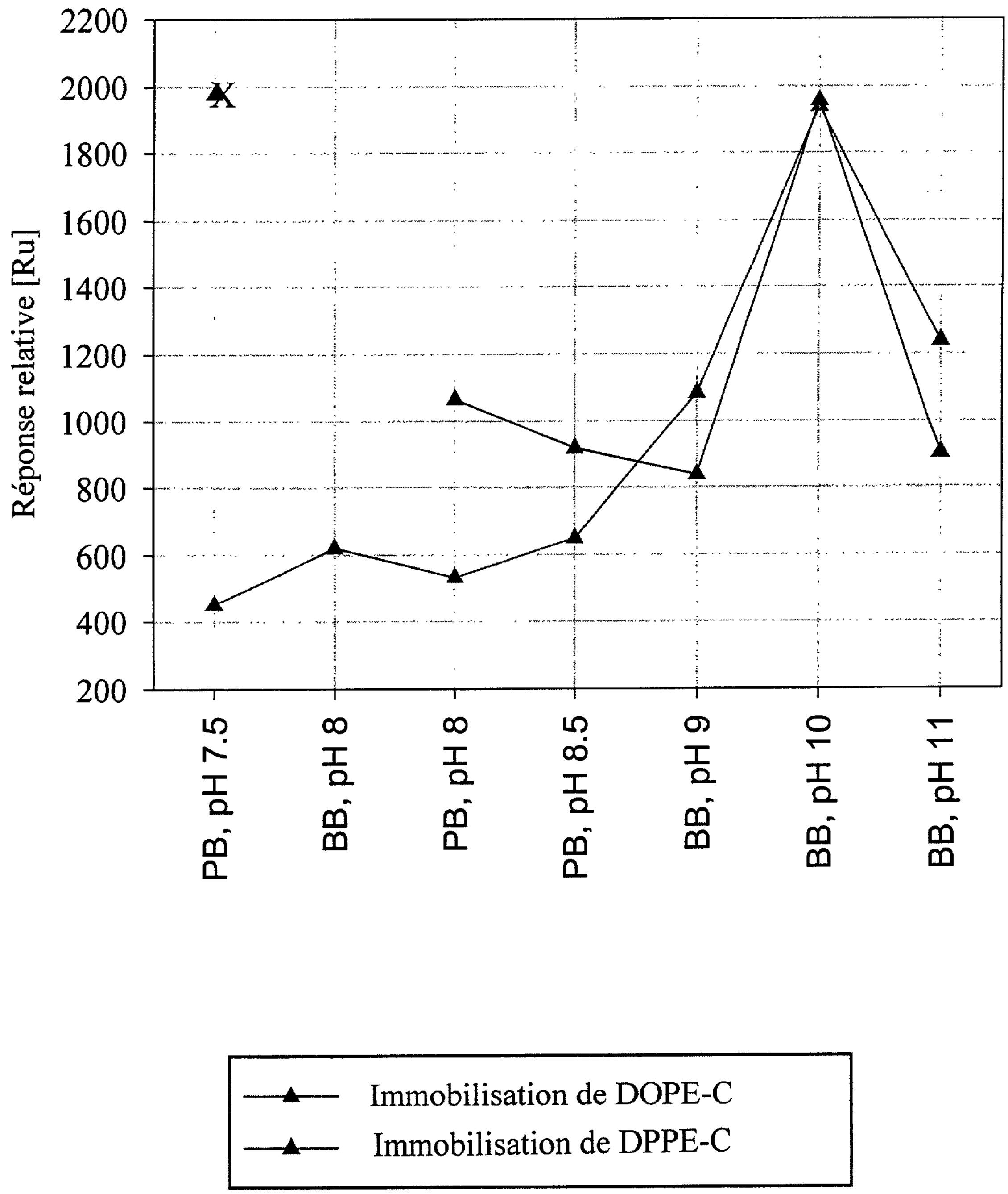
2200
2000
1800
1600
1400
1200
1000
800
600
400
200
Réponse relative [Ru]
PB, pH 7.5
BB, pH 8
PB, pH 8
PB, pH 8.5
BB, pH 9
BB, pH 10
BB, pH 11
Immobilisation de DOPE-C
Immobilisation de DPPE-C

Figure 8

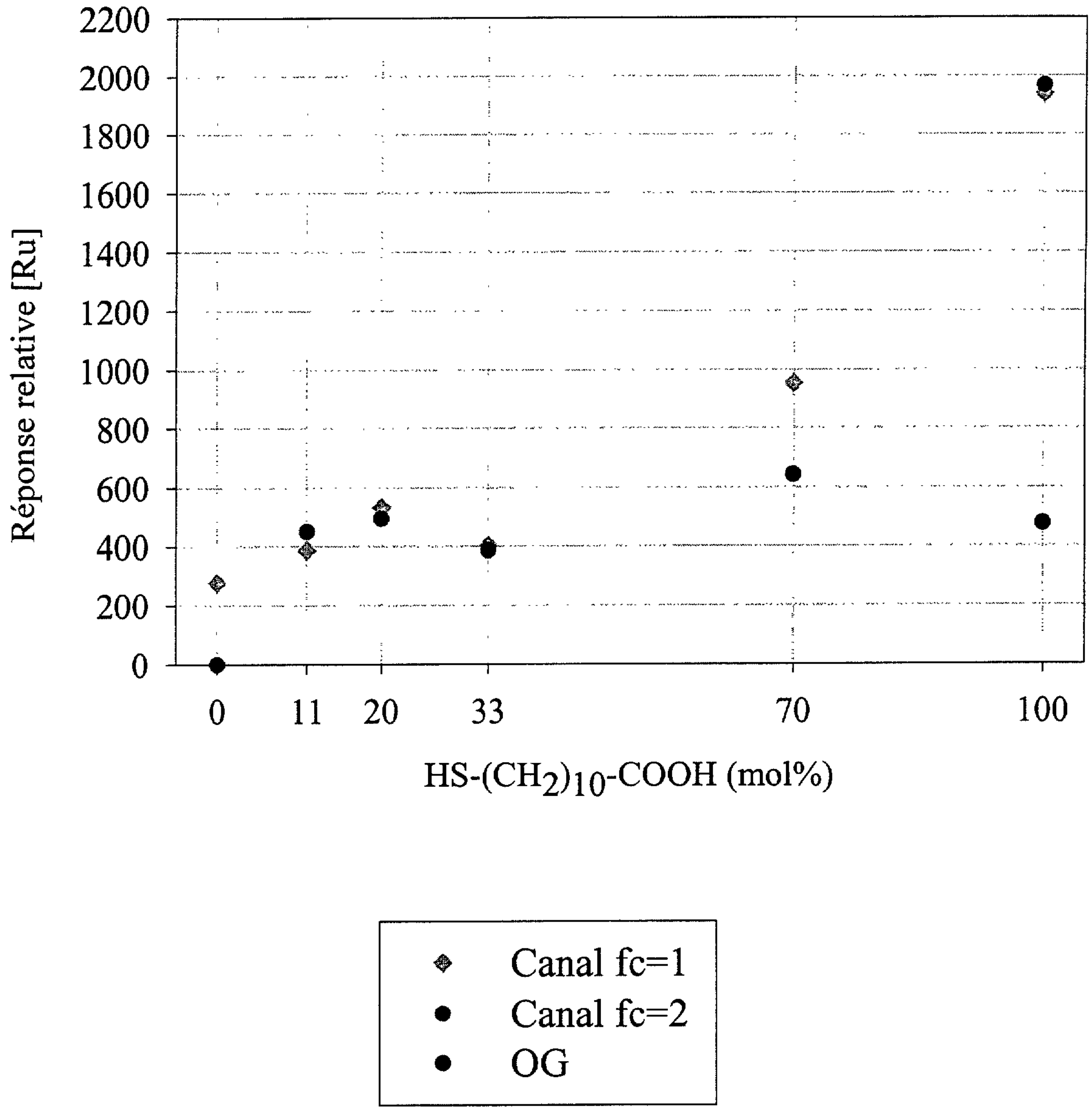
2200
2000
1800
1600
1400
1200
1000
800
600
400
200
0
Réponse relative [Ru]
0
11
20
33
70
100
HS-(CH2)10-COOH (mol%)
Canal fc=1
Canal fc=2
OG

Figure 9

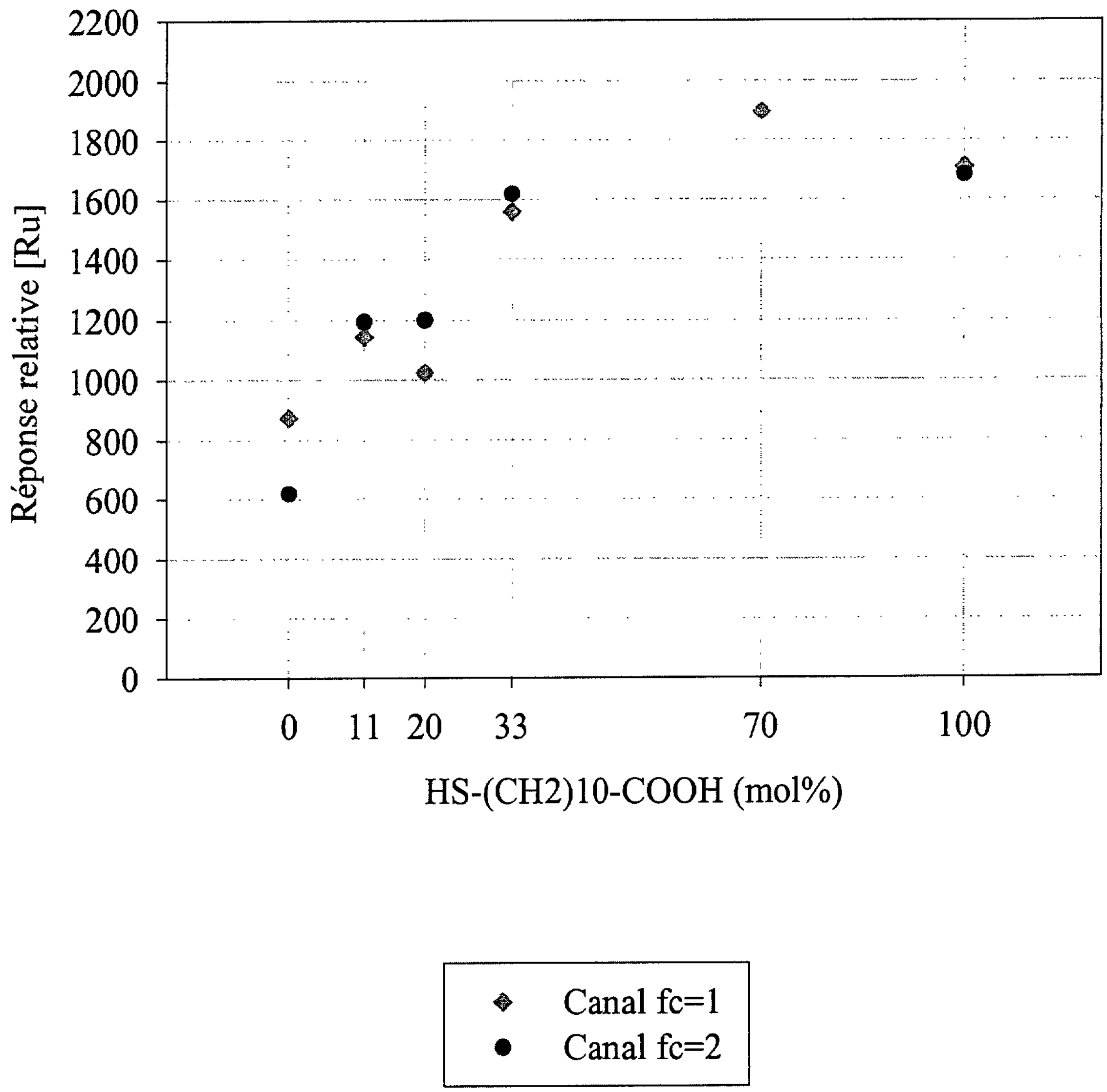

2200
2000
1800
1600
1400
1200
1000
800
600
400
200
0
Réponse relative [Ru]
0
11
20
33
70
100
HS-(CH2)10-COOH (mol%)
Canal fc=1
Canal fc=2

Figure 10

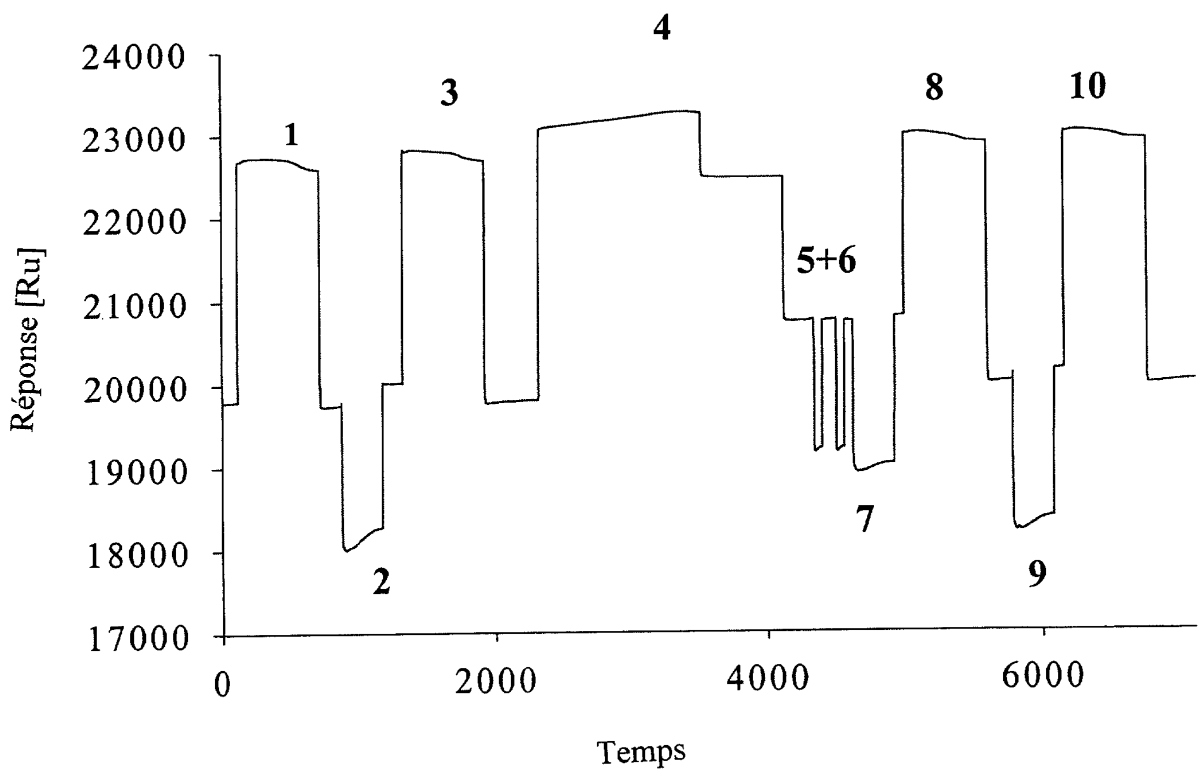
24000
23000
22000
21000
20000
19000
18000
17000
Réponse [Ru]
0
2000
4000
6000
Temps
1
2
3
4
5+6
7
8
9
10

Office européen des brevets

# RAPPORT DE RECHERCHE EUROPEENNE

Numéro de la demande
EP 02 29 1969

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | WO 96 38726 A (HEYSE STEPHAN ;VOGEL HORST (CH); ECOLE POLYTECH (CH); SAENGER MICH) 5 décembre 1996 (1996-12-05)<br>* le document en entier * | 1,7,8 | C12Q1/00<br>G01N33/543<br>G01N27/327<br>A61L27/00<br>A61L29/00 |
| A | US 5 756 355 A (VOGEL HORST ET AL) 26 mai 1998 (1998-05-26)<br>* abrégé * | 1,7,8 | |
| A | US 5 637 201 A (BRAACH-MAKSVYTIS VIJOLETA L ET AL) 10 juin 1997 (1997-06-10)<br>* abrégé * | 1,7,8 | |
| A | EP 0 441 120 A (YEDA RES & DEV) 14 août 1991 (1991-08-14)<br>* abrégé * | 1,7,8 | |

DOMAINES TECHNIQUES RECHERCHES (Int.Cl.7)

C12Q
G01N

Le présent rapport a été établi pour toutes les revendications

| Lieu de la recherche | Date d'achèvement de la recherche | Examinateur |
|---|---|---|
| LA HAYE | 8 novembre 2002 | Moreno, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 02 29 1969

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-11-2002

| Document brevet cité au rapport de recherche | | Date de publication | Membre(s) de la famille de brevet(s) | | | Date de publication |
|---|---|---|---|---|---|---|
| WO 9638726 | A | 05-12-1996 | WO | 9638726 | A1 | 05-12-1996 |
| US 5756355 | A | 26-05-1998 | AT | 143730 | T | 15-10-1996 |
| | | | DE | 69305160 | D1 | 07-11-1996 |
| | | | DE | 69305160 | T2 | 30-04-1997 |
| | | | WO | 9321528 | A1 | 28-10-1993 |
| | | | EP | 0637384 | A1 | 08-02-1995 |
| | | | JP | 7508342 | T | 14-09-1995 |
| US 5637201 | A | 10-06-1997 | AT | 210731 | T | 15-12-2001 |
| | | | AU | 672638 | B2 | 10-10-1996 |
| | | | AU | 5144493 | A | 26-04-1994 |
| | | | WO | 9407593 | A1 | 14-04-1994 |
| | | | CA | 2145996 | A1 | 14-04-1994 |
| | | | DE | 69331333 | D1 | 24-01-2002 |
| | | | DE | 69331333 | T2 | 14-08-2002 |
| | | | DK | 670751 | T3 | 08-04-2002 |
| | | | EP | 1130386 | A1 | 05-09-2001 |
| | | | EP | 1130387 | A1 | 05-09-2001 |
| | | | EP | 1130388 | A1 | 05-09-2001 |
| | | | EP | 1106998 | A2 | 13-06-2001 |
| | | | EP | 1104883 | A2 | 06-06-2001 |
| | | | EP | 0670751 | A1 | 13-09-1995 |
| | | | ES | 2169725 | T3 | 16-07-2002 |
| | | | JP | 8505123 | T | 04-06-1996 |
| | | | PT | 670751 | T | 31-05-2002 |
| | | | US | 5783054 | A | 21-07-1998 |
| | | | US | 5753093 | A | 19-05-1998 |
| | | | US | 5741409 | A | 21-04-1998 |
| EP 0441120 | A | 14-08-1991 | IL | 93020 | A | 29-06-1995 |
| | | | AT | 130938 | T | 15-12-1995 |
| | | | AU | 625017 | B2 | 25-06-1992 |
| | | | AU | 6924591 | A | 11-07-1991 |
| | | | CA | 2033776 | A1 | 10-07-1991 |
| | | | DE | 69114870 | D1 | 11-01-1996 |
| | | | DE | 69114870 | T2 | 29-08-1996 |
| | | | EP | 0441120 | A2 | 14-08-1991 |
| | | | ES | 2082867 | T3 | 01-04-1996 |
| | | | JP | 3213341 | B2 | 02-10-2001 |
| | | | JP | 6090736 | A | 05-04-1994 |
| | | | US | 5204239 | A | 20-04-1993 |

EPO FORM P0460

Pour tout renseignement concernant cette annexe : voir Journal Officiel de l'Office européen des brevets, No.12/82